# EUROPEAN PATENT APPLICATION

(11) **EP 3 831 930 A1**
(43) Date of publication of application: **09.06.2021**
(21) Application number: 20203941.8
(22) Date of filing: 21.07.2010
(51) Int. Cl.: C12N 3/00, A61K 35/42, C12N 5/077, A61P 9/00, A61P 29/00, A61K 35/12, A61P 7/00, G01N 33/50, C12N 5/074, A61P 43/00

(54) **USE OF STEM CELLS TO REDUCE LEUKOCYTE EXTRAVASATION**

(30) Priority: 21.07.2009 US 22730909 P
(62) Divisional of application: 10802840.8
(71) Applicant: ABT Holding Company, Cleveland, OH 44115 (US)
(72) Inventor: WODA, Juliana Megan, Shaker Heights, OH 44120 (US); VAN'T HOF, Wouter, Shaker Heights, OH 44118 (US)
(74) Representative: Newton, Michael Jonathan

(57) **Abstract**

The invention is generally directed to reducing inflammation by means of cells that secrete factors that reduce leukocyte extravasation. Specifically, the invention is directed to methods using cells that secrete factors that downregulate the expression of cellular adhesion molecules in vascular endothelial cells. Downregulating expression of cellular adhesion molecules reduces leukocyte adhesion to the endothelial cells such that extravasation is reduced. The end result is a reduction of inflammation. The cells are non-embryonic non-germ cells that have pluripotent characteristics. These may include expression of pluripotential markers and broad differentiation potential.

## Description

### FIELD OF THE INVENTION

The invention is generally directed to reducing inflammation by means of cells that secrete factors that reduce leukocyte extravasation. Specifically, the invention is directed to methods using cells that secrete factors that downregulate the expression of cellular adhesion molecules in vascular endothelial cells. Downregulating expression of cellular adhesion molecules reduces leukocyte adhesion to the endothelial cells such that extravasation is reduced. The end result is a reduction of inflammation. Thus, the invention provides methods for treating pathological conditions associated with an undesirable inflammatory component, including cardiovascular disease. The invention is also directed to drug discovery methods to screen for agents that modulate the ability of the cells to downregulate expression of cellular adhesion molecules in endothelial cells. The invention is also directed to cell banks that can be used to provide cells for administration to a subject, the banks comprising cells having a desired potency with respect to downregulating expression of cellular adhesion molecules in endothelial cells and reducing leukocyte adhesion and extravasation. The invention is also directed to compositions comprising cells of specific desired potencies. The invention is also directed to diagnostic methods conducted prior to administering the cells, including assays to assess the desired potency of the cells to be administered. The invention is further directed to post-treatment diagnostic assays to assess the effect of the cells on a subject being treated. The invention is also directed to cells of a desired potency in pharmaceutical compositions. The cells are non-embryonic non-germ cells that have pluripotent characteristics. These may include expression of pluripotential markers and broad differentiation potential.

### BACKGROUND OF THE INVENTION

### Inflammation

The process of acute inflammation is initiated by the blood vessels local to the injured tissue, which alter to allow the exudation of plasma proteins and leukocytes into the surrounding tissue. The increased flow of fluid into the tissue causes the characteristic swelling associated with inflammation. The blood vessels undergo marked vascular changes, including vasodilation, increased permeability, and the slowing of blood flow, which are induced by the actions of various inflammatory mediators. Increased permeability of the vessels results in the movement of plasma into the tissues, with resultant stasis due to the increase in the concentration of the cells within blood. Stasis allows leukocytes to marginate along the endothelium, a process critical to their recruitment into the tissues. Normal flowing blood prevents this, as the shearing force along the periphery of the vessels moves cells in the blood into the middle of the vessel. The changes thus permit the extravasation of leukocytes through the endothelium and basement membrane constituting the blood vessel. Once in the tissue, the cells migrate along a chemotactic gradient to reach the site of injury, where they can attempt to remove the stimulus and repair the tissue.

Leukocyte movement from the blood to the tissues through the blood vessels is known as extravasation, and can be divided up into a number of broad steps:
(1) Leukocyte localisation and recruitment to the endothelium local to the site of inflammation-involving margination and adhesion to the endothelial cells: Recruitment of leukocytes is receptor-mediated. The products of inflammation, such as histamine, promote the immediate expression of P-selectin on endothelial cell surfaces. This receptor binds weakly to carbohydrate ligands on leukocyte surfaces and causes them to "roll" along the endothelial surface as bonds are made and broken. Cytokines from injured cells induce the expression of E-selectin on endothelial cells, which functions similarly to P-selectin. Cytokines also induce the expression of integrin ligands on endothelial cells, which further slow leukocytes down. These weakly bound leukocytes are free to detach if not activated by chemokines produced in injured tissue. Activation increases the affinity of bound integrin receptors for ligands on the endothelial cell surface, firmly binding the leukocytes to the endothelium.
(2) Migration across the endothelium, known as transmigration, via the process of diapedesis: Chemokine gradients stimulate the adhered leukocytes to move between endothelial cells and pass the basement membrane into the tissues.
(3) Movement of leukocytes within the tissue via chemotaxis: Leukocytes reaching the tissue interstitium bind to extracellular matrix proteins via expressed integrins and CD44 to prevent their loss from the site. Chemoattractants cause the leukocytes to move along a chemotactic gradient towards the source of inflammation.

Leukocyte extravasation is the movement of leukocytes out of the circulatory system, towards the site of tissue damage or infection. This process forms part of the innate immune response, involving the recruitment of non-specific leukocytes. Monocytes also use this process in the absence of infection or tissue damage during their development into macrophages.

Leukocyte extravasation occurs mainly in post-capillary venules, where hemodynamic shear forces are minimized. This process can be understood in several steps, outlined below as "chemoattraction," "rolling adhesion," "tight adhesion," and "(endothelial) transmigration." It has been demonstrated that leukocyte recruitment is halted whenever any of these steps is suppressed.

### Chemoattraction

Upon recognition of and activation by pathogens, resident macrophages in the affected tissue release cytokines such as IL-1, TNF-α and chemokines. IL-1 and TNF-α cause the endothelial cells of blood vessels near the site of infection to express cellular adhesion molecules, including selectins. Circulating leukocytes are localised towards the site of injury or infection due to the presence of chemokines.

### Rolling adhesion

Like velcro, carbohydrate ligands on the circulating leukocytes bind to selectin molecules on the inner wall of the vessel, with marginal affinity. This causes the leukocytes to slow down and begin rolling along the inner surface of the vessel wall. During this rolling motion, transitory bonds are formed and broken between selectins and their ligands.

### Tight adhesion

At the same time, chemokines released by macrophages activate the rolling leukocytes and cause surface integrin molecules to switch from the default low-affinity state to a high-affinity state. This is assisted through juxtacrine activation of integrins by chemokines and soluble factors released by endothelial cells. In the activated state, integrins bind tightly to complementary receptors expressed on endothelial cells, with high affinity. This causes the immobilisation of the leukocytes, despite the shear forces of the ongoing blood flow.

### Transmigration

The cytoskeletons of the leukocytes are reorganized in such a way that the leukocytes are spread out over the endothelial cells. In this form, leukocytes extend pseudopodia and pass through gaps between endothelial cells. Transmigration of the leukocyte occurs as PECAM proteins, found on the leukocyte and endothelial cell surfaces, interact and effectively pull the cell through the endothelium. The leukocytes secrete proteases that degrade the basement membrane, allowing them to escape the blood vessel - a process known as diapedesis. Once in the interstitial fluid, leukocytes migrate along a chemotactic gradient towards the site of injury or infection.

Neutrophil accumulation at sites of inflammation is mediated by specific groups of cell adhesion molecules including the CD18 integrins on leukocytes and the selectins (P- and E-selectin on the endothelium and L-selectin on the leukocytes). This is supported by studies of patients with leukocyte adhesion deficiency syndromes whose leukocytes are genetically deficient in the expression of CD18 or selectin carbohydrate ligands (e.g., leukocyte adhesion deficiency type II.

### Selectins

Selectins are expressed shortly after cytokine activation of endothelial cells by tissue macrophages. Activated endothelial cells initially express P-selectin molecules, but within two hours after activation E-selectin expression is favored. Endothelial selectins bind carbohydrates on leukocyte transmembrane glycoproteins, including sialyl-Lewis^{x}.

P-selectins: P-selectin is expressed on activated endothelial cells and platelets. Synthesis of P-selectin can be induced by thrombin, leukotriene B4, complement fragment C5a, histamine, TNF-α or LPS. These cytokines induce the externalization of Weibel-Palade bodies in endothelial cells, presenting pre-formed P-selectins on the endothelial cell surface. P-selectins bind PSGL-1 as a ligand.

E-selectins: Endothelial leukocyte adhesion molecule-1 is expressed by cytokine-stimulated endothelial cells. It is thought to be responsible for the accumulation of blood leukocytes at sites of inflammation by mediating the adhesion of cells to the vascular lining. It exhibits structural features homologous to those of LYAM1, including the presence of lectin- and EGF-like domains followed by short consensus repeat (SCR) domains that contain 6 conserved cysteine residues. These proteins are part of the selectin family of cell adhesion molecules (Watson et al.,_J. Exp. Med. 172: 263-272 (1990); Collins et al.,_J. Biol. Chem. 266: 2466-2473 (1991)). Synthesis of E-selectin follows shortly after P-selectin synthesis, induced by cytokines such as IL-1 and TNF-α. E-selectins bind PSGL-1 and ESL-1.

L-selectins: L-selectins are constitutively expressed on some leukocytes, and are known to bind GlyCAM-1, MadCAM-1 and CD34 as ligands.

Suppressed expression of some selectins results in a slower immune response. If L-selectin is not produced, the immune response may be ten times slower, as P-selectins (which can also be produced by leukocytes) bind to each other. P-selectins can bind each other with high affinity, but occur less frequently because the receptor-site density is lower than with the smaller E-selectin molecules. This increases the initial leukocyte rolling speed, prolonging the slow rolling phase.

### Integrins

Integrins involved in cellular adhesion are primarily expressed on leukocytes. β2 integrins on rolling leukocytes bind endothelial cellular adhesion molecules, arresting cell movement.
LFA-1 is found on circulating leukocytes, and binds ICAM-1 and ICAM-2 on endothelial cells
Mac-1 is found on circulating leukocytes, and binds ICAM-1 on endothelial cells
VLA-4 is found on leukocytes and endothelial cells, and facilitates chemotaxis; it also binds VCAM-1

Cellular activation via extracellular chemokines causes pre-formed β2 integrins to be released from cellular stores. Integrin molecules migrate to the cell surface and congregate in high-avidity patches. Intracellular integrin domains associate with the leukocyte cytoskeleton, via mediation with cytosolic factors such as talin, α-actinin and vinculin. This association causes a conformational shift in the integrin's tertiary structure, allowing ligand access to the binding site. Divalent cations (e.g. Mg²⁺) are also required for integrin-ligand binding.

Integrin ligands ICAM-1 and VCAM-1 are activated by inflammatory cytokines, while ICAM-2 is constitutively expressed by some endothelial cells but downregulated by inflammatory cytokines. ICAM-1 and ICAM-2 share two homologous N-terminal domains; both can bind LFA-1.

During chemotaxis, cell movement is facilitated by the binding of β1 integrins to components of the extracellular matrix: VLA-3, VLA-4 and VLA-5 to fibronectin and VLA-2 and VLA-3 to collagen and other extracellular matrix components.

### Cytokines

Extravasation is regulated by the background cytokine environment produced by the inflammatory response, and is independent of specific cellular antigens. Cytokines released in the initial immune response induce vasodilation and lower the electrical charge along the vessel's surface. Blood flow is slowed, facilitating intermolecular binding.
IL-1 activates resident lymphocytes and vascular endothelia
TNF-α increases vascular permeability and activates vascular endothelia
CXCL8 (IL-8) forms a chemotactic gradient that directs leukocytes towards site of tissue injury/infection (CCL2 has a similar function to CXCL8, inducing monocyte extravasation and development into macrophages); also activates leukocyte integrins.

Review articles summarizing the extravasation process in inflammation are available. See Steeber, D. and Tedder, T., Immunologic Research, 22/2-3:299-317 (2000); Steeber et al., FSAEB J, 9:866-873 (1995); and Wagner, D. and Frenette, P., Blood, 111:5271-5281 (2008).

CD15 (3-fucosyl-N-acetyl-lactosamine) is a cluster of differentiation antigen - an immunologically significant molecule. CD15 is a carbohydrate adhesion molecule (not a protein) that can be expressed on glycoproteins, glycolipids and proteoglycans.

CD15 mediates phagocytosis and chemotaxis, found on neutrophils; expressed in patients with Hodgkin disease, some B-cell chronic lymphocytic leukemias, acute lymphoblastic leukemias, and most acute non-lymphocytic leukemias. It is also called Lewis x and SSEA-1 (stage specific embryonic antigen 1) and represents a marker for murine pluripotent stem cells, in which it plays an important role in adhesion and migration of the cells in the preimplantation embryo. It is synthesized by FUT4 (fucosyltransferase 4) and FUT9.

### CD15s

The sialyl Lewis x oligosaccharide determinant is an essential component of leukocyte counter-receptors for E-selectin and P-selectin-mediated adhesions of leukocytes. This oligosaccharide molecule is displayed on the surfaces of granulocytes, monocytes, and natural killer cells. Formation of leukocyte adhesion to these selectins is an early and important step in the process that ultimately allows leukocytes to leave the vascular tree and become recruited into lymphoid tissues and sites of inflammation. Natsuka et al., J. Biol. Chem. 269: 16789-16794 (1994) and Sasaki et al., J. Biol. Chem. 269: 14730-14737 (1994) isolated cDNAs encoding a human leukocyte alpha-1,3-fucosyltransferase, FUT7, capable of synthesizing the sialyl Lewis x determinant.

Natsuka et al. found when FUT7 was expressed in mammalian cells, the cDNA directed synthesis of cell surface sialyl Lewis x moieties, but not Lewis x, Lewis A, sialyl Lewis a, or VIM-2 determinants. Sasaki et al. demonstrated in vivo ability of FUT7 to synthesize the sialyl Lewis x moiety that binds to E-selectin and reported the restricted expression of FUT7 in leukocytes.

Chen et al., Proc. Nat. Acad. Sci. 103:16894-16899 (2006) noted that activated T cells, particularly Th1 cells, express sialyl Lewis x, but resting T cells do not. Using reporter analysis, they showed that TBET promoted and GATA3 repressed transcription of FUT7. TBET interfered with GATA3 binding to its target DNA, but GATA3 also interfered with TBET binding to the FUT7 promoter. GATA3 regulated FUT7 transcription by recruiting, in a phosphorylation-dependent manner, histone deacetylase-3 and HDAC5 and by competing with CBP/p300 in binding to the N terminus of TBET. Maximal expression of FUT7 and sialyl Lewis x in T cells was obtained by ROG-mediated suppression of GATA3. Chen et al. (2006) concluded that the GATA3/TBET transcription factor complex regulates cell lineage-specific expression of lymphocyte homing receptors and that glycoconjugates are regulated by this complex to attain cell lineage-specific expression in Th1 and Th2 lymphocyte subsets.

Selectin P ligand, or P-selectin glycoprotein ligand (PSGL1), is the high affinity counter-receptor for P-selectin on myeloid cells and stimulated T lymphocytes. As such, it plays a critical role in the tethering of these cells to activated platelets or endothelia expressing P-selectin.

Based on flow cytometry, immunoblot, and flow chamber analyses, Fuhlbrigge et al.,_Nature 389: 978-981 (1997) proposed that a differential posttranslational modification of PSGL1, mediated by fucosyltransferase-7, regulates the expression of the cutaneous lymphocyte-associated antigen (CLA), which binds both P-selectin and E-selectin, in T cells. CLA-positive T cells are skin-homing memory T cells that are defined by their reactivity with monoclonal antibody HECA-452. CLA-positive T cells infiltrate skin lesions in a number of inflammatory skin disorders, including psoriasis.

### SUMMARY OF THE INVENTION

The invention is broadly directed to a method for reducing inflammation.

The invention is more specifically directed to a method to reduce extravasation of leukocytes (neutrophils, lymphocytes, and monocytes).

The invention is more specifically directed to a method to reduce leukocyte infiltration from the circulatory system into surrounding tissues.

The invention is also directed to a method to reduce adhesion of leukocytes to a blood vessel.

The invention is also directed to a method to reduce leukocyte adhesion to vascular endothelium.

The invention is also directed to a method to reduce leukocyte adhesion to endothelial cells in the blood vessel wall.

The invention is also directed to a method for downregulating expression of adhesion molecules in endothelial cells.

The invention is also directed to a method to reduce endothelial cell activation.

The invention is also directed to a method to reduce endothelial cell activation in the vascular endothelium.

Endothelial cell activation may result from exposure to an inflammatory cytokine. Cytokines include, but are not limited to, tumor necrosis factor-α (TNF-α) interleukin-1 (IL-1), amphiregulin, LPS and other Toll-like receptor ligands (pathogenic peptides such as fMLP, peptides from damaged tissues, such as fibronectin fragments) thrombin, histamines, oxygen radicals, and IFN-γ. In the context of the invention, activation involves upregulation of cell adhesion molecules in vascular endothelial cells.

The invention is also directed to a method to reduce the ability of endothelial cells in the blood vessel wall to bind to an adhesion ligand or moiety on a leukocyte. These include, but are not limited to, PSGL-1, ESL-1, CD15s, α4-integrins, CD44, β2-integrins, L-selectin, CD99, and JAMs.

Leukocytes include, but are not limited to, neutrophils, monocytes, and lymphocytes. Lymphocytes include B-cells and T-cells. T-cells include CD4⁺, CD8⁺, γδT cells, and natural killer cells.

Cellular adhesion molecules expressed in endothelial cells and subject to downregulation include, but are not limited to, E-selectin VCAM, ICAM (1, 2), P-selectin, CD99, PECAM-1, JAMs, ESAM, and osteopontin (co-receptor for VCAM).

In one embodiment, I-CAM and P-selectin are upregulated on endothelial cells in a subject having a traumatic brain injury.

The invention is directed to reducing cellular adhesion molecule expression. Expression can be both intracellular and extracellular, such as on the endothelial cell surface. Accordingly, expression may be reduced such that there is a reduction of secretion of these molecules into cell culture medium when endothelial cells are in culture. Extracellular and cell surface expression includes reducing expression at the protein level. Intracellular expression includes reducing both transcription and translation within the cell.

According to this invention, the reduction of all of the above effects (i.e., inflammation, extravasation, leukocyte adhesion to endothelial cells, expression of adhesion molecules, etc.) are achieved by means of exposing the leukocytes and/or endothelial cells to a non-embryonic non-germ cell having some of the pluripotential characteristics of an embryonic stem cell but derived from non-embryonic tissue.

According to this invention, all of the above effects can be achieved by administering cells or medium conditioned by the cells. Cells include, but are not limited to, non-embryonic non-germ cells having characteristics of embryonic stem cells, but being derived from non-embryonic tissue. Such cells may be pluripotent and express pluripotency markers, such as one or more of oct4, telomerase, rex-1, rox-1, sox-2, nanog, SSEA-1 and SSEA-4. Other characteristics of pluripotency can include the ability to differentiate into cell types of more than one germ layer, such as two or three of ectodermal, endodermal, and mesodermal embryonic germ layers. Such cells may or may not be immortalized or transformed in culture. Such cells may be highly expanded without being transformed and also maintain a normal karyotype. For example, in one embodiment, the non-embryonic non-germ cells may have undergone at least 10-40 cell doublings, such as 30, 40, 50, 60, or more, wherein the cells are not transformed and have a normal karyotype. The cells may express telomerase activity so as to be able to achieve more than 30 population doublings (cell doublings), such as 35, 40, 45, 50, or more. However, as noted above, the cells could further express one or more of oct4, telomerase, rex-1, rox-1, sox-2, nanog, SSEA1, or SSEA4. Such cells may differentiate into at least one cell type of each of two of the endodermal, ectodermal, and mesodermal embryonic lineages and may include differentiation into all three. Further, they may or may not be tumorigenic, such as not producing teratomas. If cells are transformed or tumorigenic, and it is desirable to use them for infusion, such cells may be disabled so they cannot form tumors *in vivo,* as by treatment that prevents cell proliferation into tumors. Such treatments are well known in the art. Such cells may naturally achieve the effects herein (i.e., not genetically or pharmaceutically modified to do this). However, natural expressors can be genetically or pharmaceutically modified to increase potency.

In view of the property of these cells to achieve the above effects, the cells can be used in drug discovery methods to screen for an agent that modulates the ability of the cells to achieve any of the above effects. Such agents include, but are not limited to, small organic molecules, antisense nucleic acids, siRNA DNA aptamers, peptides, antibodies, non-antibody proteins, cytokines, chemokines, and chemo-attractants. Then the agent can be used to increase potency of the cells to achieve any of the above effects.

As an example, this assay has been used to identify a pre-treatment of the cells with TNF-α, IFN-γ, or IL-1β, or a combination thereof, to produce cells that inhibit upregulation of the endothelial cell molecules or downregulate expression of these molecules. Some cells must be co-cultured with activated endothelial cells in order to produce the factors that downregulate expression of the adhesion molecules (or inhibit upregulation). Accordingly, naive cells (not exposed to activated endothelial cells) can be exposed to desired agents and then assayed for their effect on endothelial cell activation, expression of adhesion moieties, or any of the above effects.

Because the effects described in this application can be caused by factors secreted from the cells, not only the cells, but conditioned medium produced from culturing the cells, is useful to achieve the effects. Such medium would contain the secreted factors and, therefore, could be used instead of the cells or added to the cells. So, where cells can be used, it should be understood that medium would also be effective and could be substituted or added.

In view of the property of these cells to achieve the above effects, cells banks can be established containing cells that are selected for having a desired potency for achieving the above effects. Accordingly, the invention encompasses assaying such cells for the ability to achieve any of the above effects and selecting for and banking cells having a desired potency. The bank provides a source for making a pharmaceutical composition to administer to the subject. Cells can be used directly from the bank or expanded prior to use.

Accordingly, the invention also is directed to diagnostic procedures conducted prior to administering these cells to a subject, the pre-diagnostic procedures including assessing the potency of the cells to achieve one or more of the above effects. The cells may be taken from a cell bank and used directly or expanded prior to administration. In either case, the cells would be assessed for the desired potency. Or the cells can be derived from the subject and expanded prior to administration. In this case, as well, the cells would be assessed for the desired potency prior to administration.

Although the cells selected for effectiveness are necessarily assayed during the selection procedure, it may be preferable and prudent to again assay the cells prior to administration to a subject for treatment to ensure that the cells still are effective at desired levels. This is particularly preferable where the cells have been stored for any length of time, such as in a cell bank, where cells are most likely frozen during storage.

With respect to methods of treatment with the cells, between the original isolation of the cells and the administration to a subject, there may be multiple (i.e., sequential) assays for modulation. This is to ensure that the cells can still achieve the effect at desired levels after manipulations that occur within this time frame. For example, an assay may be performed after each expansion of the cells. If cells are stored in a cell bank, they may be assayed after being released from storage. If they are frozen, they may be assayed after thawing. If the cells from a cell bank are expanded, they may be assayed after expansion. Preferably, a portion of the final cell product (that is physically administered to the subject) may be assayed.

The invention is also directed to a method for establishing the dosage of such cells by assessing the potency of the cells to achieve one or more of the above effects.

The invention further includes post-treatment diagnostic assays, following administration of the cells to assess efficacy. The diagnostic assays include, but are not limited to any of the effects described herein that are amenable to assay from a subject.

The invention is also directed to compositions comprising a population of the cells having a desired potency. Such populations may be found as pharmaceutical compositions suitable for administration to a subject and/or in cell banks from which cells can be used directly for administration to a subject or expanded prior to administration.

The methods and compositions of the invention are useful for treating any disease involving inflammation, where a component of that inflammation involves leukocyte adhesion to vascular endothelial cells by means of cellular adhesion molecules. This includes acute and chronic conditions in cardiovascular, e.g., acute myocardial infarction; central nervous system injury, e.g., stroke, traumatic brain injury, spinal cord injury; peripheral vascular disease; pulmonary, e.g., asthma, ARDS; autoimmune, e.g., rheumatoid arthritis, multiple sclerosis, lupus, sclerodoma; psoriasis; gastrointestinal, e.g., graft-versus-host-disease, Crohn's disease.

It is understood, however, that for treatment of any of the above conditions, it may be expedient to use such cells; that is, one that has been assessed for one or more of the effects described herein and selected for a desired level of effectiveness prior to administration for treatment of the condition.

### BRIEF DESCRIPTION OF THE FIGURES

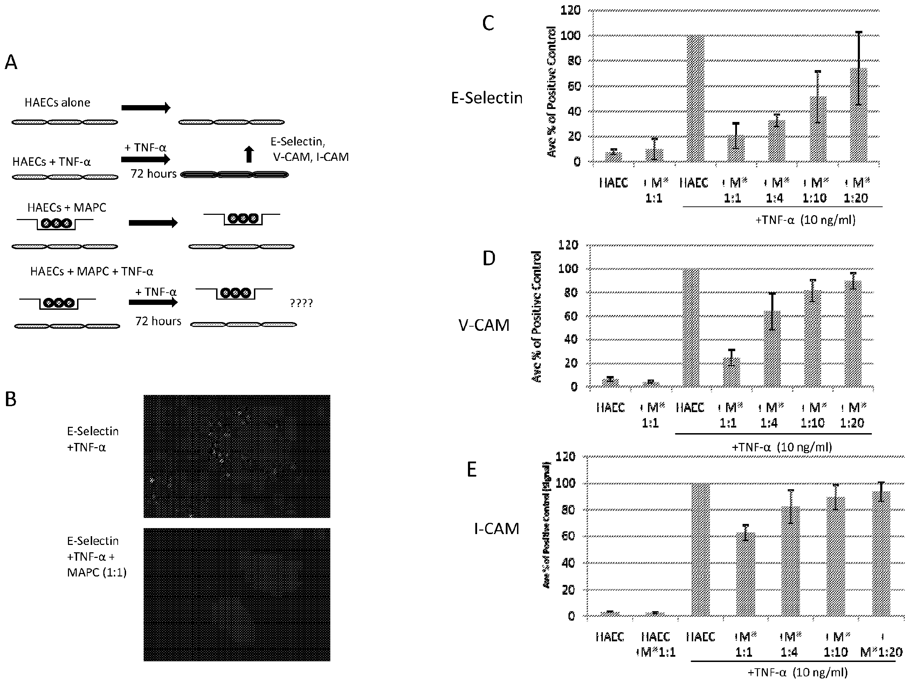

Figure 1: MultiStem modulates E-selectin, V-CAM and I-CAM upregulation by TNF- α in Human Aortic Endothelial Cells (HAEC). **(A)** After growing cells to near confluence, endothelial cells were activated with TNF-α for 72 hours alone or in co-culture with MultiStem. Cells were then analyzed by flow cytometry or PCR for expression of E-Selectin, V-CAM or I-CAM. **(B-E)** TNF-α induced endothelial cell adhesion molecule surface expression is decreased in the presence of MultiStem in a cell dose dependent manner. HAEC show reduced cell surface upregulation of E-selectin **(B,C),** V-CAM **(D)** and I-CAM **(E)** expression when co-cultured with MultiStem in the presence of TNF-α (10 ng/ml) for 72 hours. The number of cells expressing E-selectin and V-CAM was determined and expressed as a percentage of the total number of cells induced to express these markers with TNF-α. For I-CAM, all cells had some level expression even prior to activation. Therefore, the strength of signal per cell was calculated and the data is expressed as a percentage of the TNF-α induced signal.

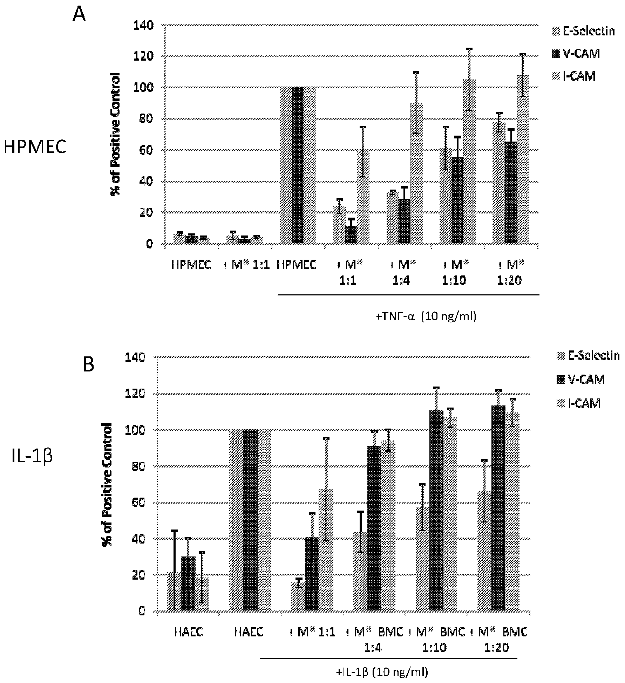

Figure 2: MultiStem inhibits cell surface adhesion molecule upregulation in human pulmonary endothelial cells (HPMEC) or by IL-1β **(A)** HPMEC show reduced cell surface upregulation of E-Selectin, V-CAM, and I-CAM expression when co-cultured with MultiStem in the presence of TNF-α (10 ng/ml) for 72 hours, similar to HAEC. Cell surface expression was measured by FACS. (B) Induction of cell surface markers by another pro-inflammatory cytokine, IL-1β, was also reduced in HAEC by co-culture with MultiStem.

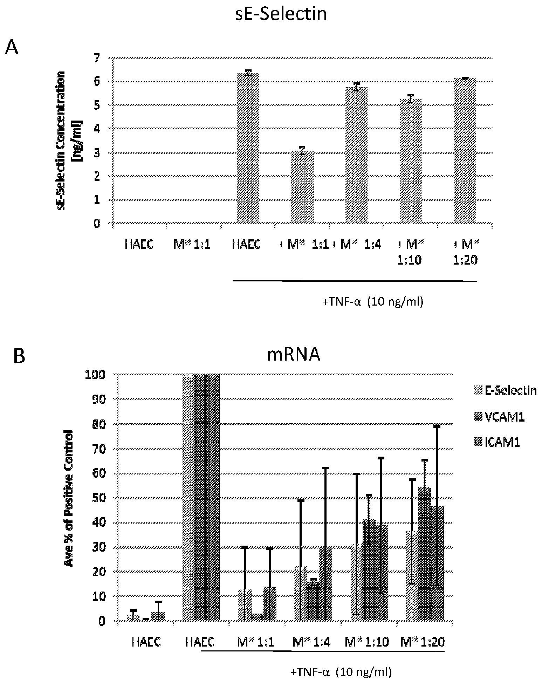

Figure 3: MultiStem regulates cell adhesion molecule upregulation at the transcriptional level (A) Levels of soluble E-selectin (sE-selectin) were measured in the media from co-cultures of MultiStem and endothelial cells (HAEC or HPMEC) by ELISA. **(B)** mRNA was isolated from HAEC after 72 hour co-culture in the absence or presence of TNF-α with differing cell doses of MultiStem. Quantitative RT-PCR was performed for E-selectin, V-CAM, I-CAM and GAPDH. MultiStem reduced the mRNA expression of TNF-α induced E-selectin, V-CAM and I -CAM compared to untreated control cells.

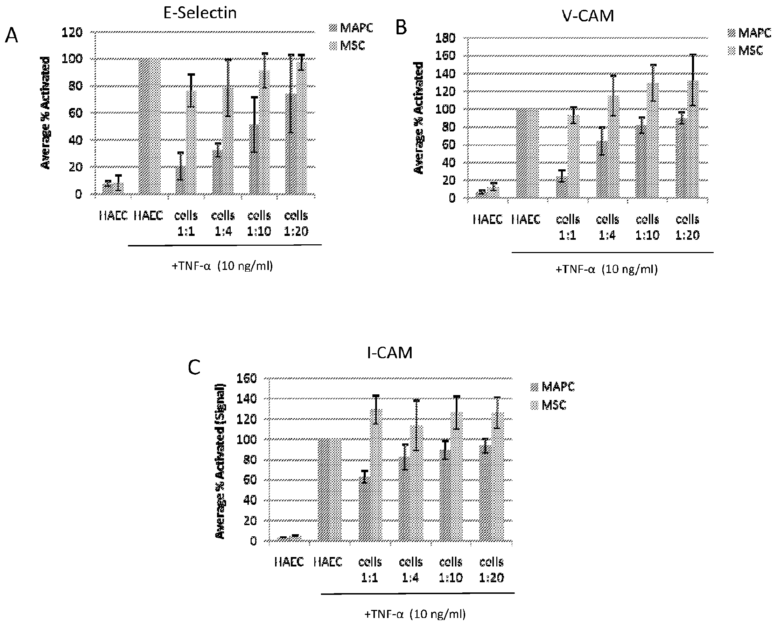

Figure 4: Unlike MultiStem, MSC do not inhibit cell surface expression of adhesion molecules in endothelial cells upon activation with TNF-α. **(A-C)** TNF-α induced endothelial cell adhesion molecule surface expression is unchanged in the presence of MSC, whereas co-culture with MultiStem reduces adhesion molecule expression. HAEC show no change in cell surface upregulation of E-selectin, V-CAM and I-CAM expression when co-cultured with MSC in the presence of TNF- α (10 ng/ml) for 72 hours.

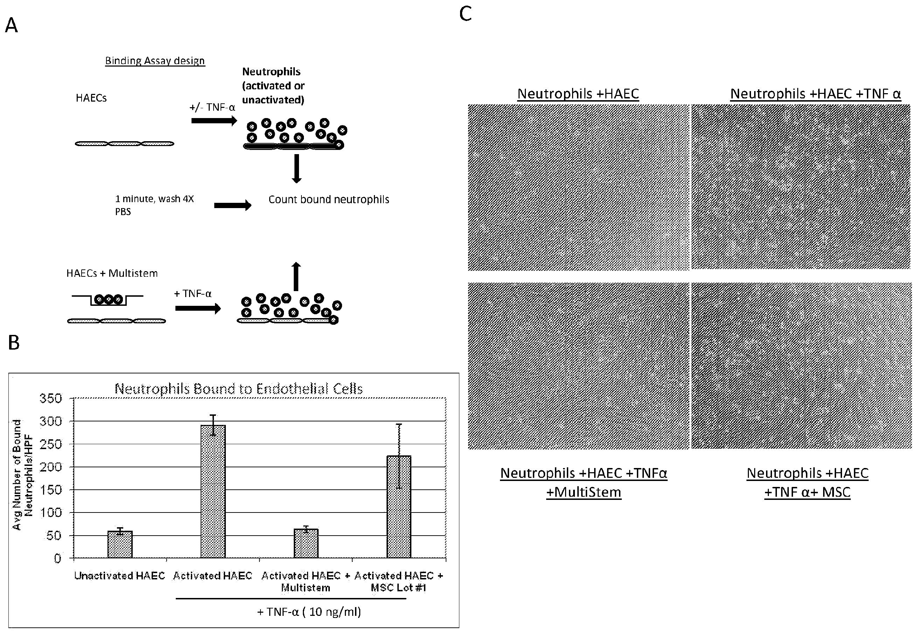

Figure 5: Neutrophil adhesion to endothelial cells co-cultured in the presence of TNF-α and MultiStem is reduced compared to binding to endothelial cells incubated with TNF-α alone. (A) After 72 hour incubation alone or with MultiStem, activated and inactivated endothelial cells were incubated with activated neutrophils for 1 minute. Cells were then washed, and photographed. The number of bound neutrophils was determined by photographing each condition in triplicate and counting the number of neutrophils bound in each high power field (10X). **(B)** The number of neutrophils bound to MultiStem co-cultured endothelial cells was significantly reduced compare to control endothelial cells. **(C)** Representative photographs illustrating decreased neutrophil binding to MultiStem co-culture endothelial cells.

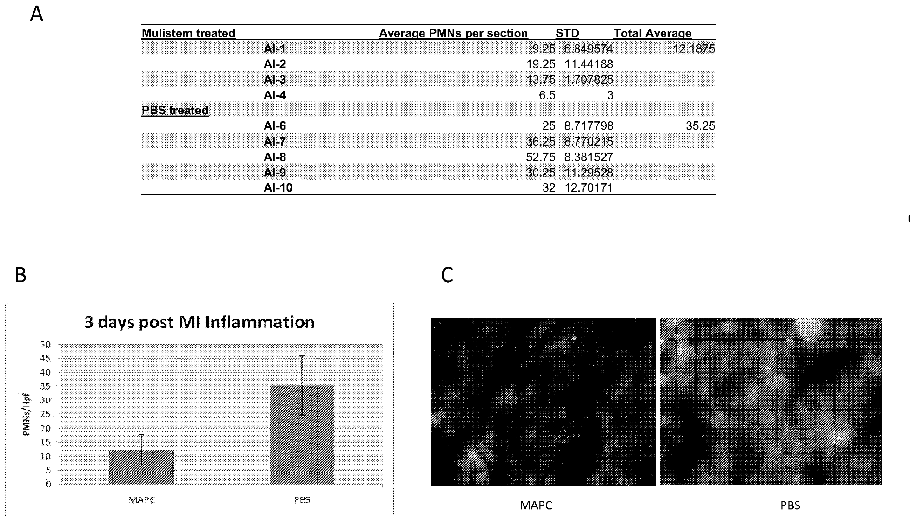

Figure 6: Neutrophil infiltration is significantly reduced in MultiStem treated hearts compared with vehicle treated hearts three days following AMI. **(A)** Acute myocardial infarction was induced by permanent LAD ligation. All rats received AMI followed by direct injection of either vehicle control (PBS) or rat MultiStem (10 Million cells). Neutrophil infiltration was measured by cells exhibiting elastase staining in heart sections from treated and untreated animals. Table 1 shows the average number of elastase positive cells from each animal (4 sections were examined per animal). **(B)** Neutrophil infiltration was measured by elastase staining. The control group had significantly higher levels of elastase staining (35.25 PMS/hpf) compared with MultiStem treated animals (12.185 PMN/hpf) three days following surgery (p=0.005823) (C). Representative views of neutrophil levels (as measure by elastase staining) in MultiStem treated animals and untreated animals.

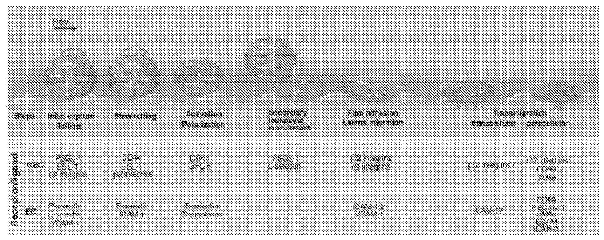

Figure 7: Multiple sequential steps mediating leukocyte recruitment during inflammation. Leukocytes are captured and begin to roll on P- and E-selectins and their ligands P-selectin glycoprotein ligand-1 (PSGL-1) and E-selectin ligand-1 (ESL-1). Some leukocytes such as lymphocytes or hematopoietic stem and progenitor cells also roll on α4 integrin and its endothelial receptor vascular cell adhesion molecule-1 (VCAM-1). L-selectin is critical for lymphocyte rolling on HEVs in lymphoid tissues. As inflammation progresses, leukocyte rolling velocity decreases, allowing the integration of activation signals from selectin ligands and G-protein-coupled receptors (GPCRs). These activation signals lead to the polarization of slowly rolling leukocytes and clustering of L-selectin and PSGL-1 to a major pole that allows further leukocyte recruitment through secondary tethers via leukocyte-leukocyte interactions. Leukocyte activation enhances integrin affinity and avidity, leading to firm adhesion on intercellular adhesion molecule-1 (ICAM-1) expressed on endothelial cells. Adherent leukocytes continuously migrate laterally to survey the microvasculature and search for possible sites for transmigration. Leukocytes can transmigrate classically through the junctional (paracellular) pathways via interactions among junctional adhesion molecules (JAMs), CD99 and platelet/endothelial-cell adhesion molecule-1 (PECAM-1), endothelial cell-selective adhesion molecule (ESAM), or alternatively through the endothelial cell (transcellular pathway.)

### DETAILED DESCRIPTION OF THE INVENTION

It should be understood that this invention is not limited to the particular methodology, protocols, and reagents, etc., described herein and, as such, may vary. The terminology used herein is for the purpose of describing particular embodiments only, and is not intended to limit the scope of the disclosed invention, which is defined solely by the claims.

The section headings are used herein for organizational purposes only and are not to be construed as in any way limiting the subject matter described.

The methods and techniques of the present application are generally performed according to conventional methods well-known in the art and as described in various general and more specific references that are cited and discussed throughout the present specification unless otherwise indicated. See, e.g., Sambrook et al., Molecular Cloning: A Laboratory Manual, 3rd ed., Cold Spring Harbor Laboratory Press, Cold Spring Harbor, N.Y. (2001) and Ausubel et al., Current Protocols in Molecular Biology, Greene Publishing Associates (1992), and Harlow and Lane, Antibodies: A Laboratory Manual, Cold Spring Harbor Laboratory Press, Cold Spring Harbor, N.Y. (1990).

### Definitions

"A" or "an" means herein one or more than one; at least one. Where the plural form is used herein, it generally includes the singular.

As used herein, the terms "adhere(s), adherence, adhesion", and the like, refer, when *in vivo,* to an association of leukocytes and endothelial cells sufficient to result in extravasation. Within the context of the invention, the adherence that is reduced or prevented by the reagents of the invention is that which occurs with such sufficiency. In *in vitro* applications, the degree (avidity) adherence may not necessarily be at that level. For example, in the context of drug discovery, one might desire to detect adhesion (binding) with an avidity that is of a lower order.

A "cell bank" is industry nomenclature for cells that have been grown and stored for future use. Cells may be stored in aliquots. They can be used directly out of storage or may be expanded after storage. This is a convenience so that there are "off the shelf' cells available for administration. The cells may already be stored in a pharmaceutically-acceptable excipient so they may be directly administered or they may be mixed with an appropriate excipient when they are released from storage. Cells may be frozen or otherwise stored in a form to preserve viability. In one embodiment of the invention, cell banks are created in which the cells have been selected for enhanced potency to achieve one or more of the effects, such as reducing expression of one or more adhesion molecules. Following release from storage, and prior to administration to the subject, it may be preferable to again assay the cells for potency. This can be done using any of the assays, direct or indirect, described in this application or otherwise known in the art. Then cells having the desired potency can then be administered to the subject for treatment.

"Co-administer" means to administer in conjunction with one another, together, coordinately, including simultaneous or sequential administration of two or more agents.

"Comprising" means, without other limitation, including the referent, necessarily, without any qualification or exclusion on what else may be included. For example, "a composition comprising x and y" encompasses any composition that contains x and y, no matter what other components may be present in the composition. Likewise, "a method comprising the step of x" encompasses any method in which x is carried out, whether x is the only step in the method or it is only one of the steps, no matter how many other steps there may be and no matter how simple or complex x is in comparison to them. "Comprised of and similar phrases using words of the root "comprise" are used herein as synonyms of "comprising" and have the same meaning.

"Comprised of" is a synonym of comprising (see above).

"Conditioned cell culture medium" is a term well-known in the art and refers to medium in which cells have been grown. Herein this means that the cells are grown for a sufficient time to secrete the factors that are effective to achieve any of the results described in this application, including reducing expression of cellular adhesion molecules; reducing the adhesion of leukocytes to endothelial cells; reducing extravasation, etc.

Conditioned cell culture medium refers to medium in which cells have been cultured so as to secrete factors into the medium. For the purposes of the present invention, cells can be grown through a sufficient number of cell divisions so as to produce effective amounts of such factors so that the medium reduces expression of cellular adhesion molecules so as to reduce adhesion of leukocytes and, hence, reduce extravasation, etc. Cells are removed from the medium by any of the known methods in the art, including, but not limited to, centrifugation, filtration, immunodepletion (e.g., via tagged antibodies and magnetic columns), and FACS sorting.

"Decrease" or "reduce" means to lower the effect or prevent it entirely, such as reduce leukocyte extravasation, adhesion, expression of adhesion moiety, or any of the effects described herein.

"EC cells" were discovered from analysis of a type of cancer called a teratocarcinoma. In 1964, researchers noted that a single cell in teratocarcinomas could be isolated and remain undifferentiated in culture. This type of stem cell became known as an embryonic carcinoma cell (EC cell).

"Effective amount" generally means an amount which provides the desired local or systemic effect, e.g., effective to ameliorate undesirable effects of inflammation by affecting adhesion that leads to extravasation. For example, an effective amount is an amount sufficient to effectuate a beneficial or desired clinical result. The effective amounts can be provided all at once in a single administration or in fractional amounts that provide the effective amount in several administrations. The precise determination of what would be considered an effective amount may be based on factors individual to each subject, including their size, age, injury, and/or disease or injury being treated, and amount of time since the injury occurred or the disease began. One skilled in the art will be able to determine the effective amount for a given subject based on these considerations which are routine in the art. As used herein, "effective dose" means the same as "effective amount."

"Effective route" generally means a route which provides for delivery of an agent to a desired compartment, system, or location. For example, an effective route is one through which an agent can be administered to provide at the desired site of action an amount of the agent sufficient to effectuate a beneficial or desired clinical result.

"Embryonic Stem Cells (ESC)" are well known in the art and have been prepared from many different mammalian species. Embryonic stem cells are stem cells derived from the inner cell mass of an early stage embryo known as a blastocyst. They are able to differentiate into all derivatives of the three primary germ layers: ectoderm, endoderm, and mesoderm. These include each of the more than 220 cell types in the adult body. The ES cells can become any tissue in the body, excluding placenta. Only the morula's cells are totipotent, able to become all tissues and a placenta. Some cells similar to ESCs may be produced by nuclear transfer of a somatic cell nucleus into an enucleated fertilized egg.

"Extravasation" refers to the leakage of a fluid out of its container. In the case of inflammation, it refers to the movement of white blood cells from the capillaries to the tissues surrounding them. This is also discussed in the Background of the Invention.

Use of the term "includes" is not intended to be limiting.

Increase" or "increasing" means to induce entirely where there was no pre-existing effect or to increase the degree of the effect, such as leukocyte extravasation, adhesion, expression of adhesion moiety, or any of the effects described herein..

"Induced pluripotent stem cells (IPSC or IPS cells)" are somatic cells that have been reprogrammed. for example, by introducing exogenous genes that confer on the somatic cell a less differentiated phenotype. These cells can then be induced to differentiate into less differentiated progeny. IPS cells have been derived using modifications of an approach originally discovered in 2006 (Yamanaka, S. et al., Cell Stem Cell, 1:39-49 (2007)). For example, in one instance, to create IPS cells, scientists started with skin cells that were then modified by a standard laboratory technique using retroviruses to insert genes into the cellular DNA. In one instance, the inserted genes were Oct4, Sox2, Lif4, and c-myc, known to act together as natural regulators to keep cells in an embryonic stem cell-like state. These cells have been described in the literature. See, for example, Wernig et al., PNAS, 105:5856-5861 (2008); Jaenisch et al., Cell, 132:567-582 (2008); Hanna et al., Cell, 133:250-264 (2008); and Brambrink et al., Cell Stem Cell, 2:151-159 (2008). These references are incorporated by reference for teaching IPSCs and methods for producing them. It is also possible that such cells can be created by specific culture conditions (exposure to specific agents).

The term "isolated" refers to a cell or cells which are not associated with one or more cells or one or more cellular components that are associated with the cell or cells *in vivo.* An "enriched population" means a relative increase in numbers of a desired cell relative to one or more other cell types *in vivo* or in primary culture.

However, as used herein, the term "isolated" does not indicate the presence of only stem cells. Rather, the term "isolated" indicates that the cells are removed from their natural tissue environment and are present at a higher concentration as compared to the normal tissue environment. Accordingly, an "isolated" cell population may further include cell types in addition to stem cells and may include additional tissue components. This also can be expressed in terms of cell doublings, for example. A cell may have undergone 10, 20, 30, 40 or more doublings *in vitro* or *ex vivo* so that it is enriched compared to its original numbers *in vivo* or in its original tissue environment (e.g., bone marrow, peripheral blood, adipose tissue, etc.).

"MAPC" is an acronym for "multipotent adult progenitor cell." In this application, the term is used to designate a cell type, namely, a non-embryonic stem cell with characteristics of an embryonic stem cell. It may give rise to cell lineages of more than one germ layer, such as two or all three germ layers (i.e., endoderm, mesoderm and ectoderm) upon differentiation. MAPCs may express one or more of telomerase, Oct 3/4 (i.e., Oct 3A), rex-1, rox-1 and sox-2, and SSEA-4. The term "adult" in MAPC is non-restrictive. It refers to a non-embryonic somatic cell. MAPCs are karyotypically normal and do not form teratomas *in vivo.* This acronym was first used in PCT/US2000/21387 to describe a pluripotent cell isolated from bone marrow. However, subsequent to isolation of these cells from bone marrow, other cells with pluripotential markers and/or differentiation potential have been discovered and, for purposes of this invention, may be functionally equivalent, with respect to the effects described herein, to those cells first designated "MAPC."

The term "MultiStem®" is the trade name for a non-embryonic non-germ cell that is highly expandable, karyotypically normal, and does not form teratomas *in vivo.* It may differentiate into cell lineages of more than one germ layer. The cells may express one or more of telomerase, oct3/4, rex-1, rox-1, sox-2, and SSEA4. MultiStem® is prepared according to cell culture methods disclosed in this patent application, in particular, lower oxygen and higher serum.

"Pharmaceutically-acceptable carrier" is any pharmaceutically-acceptable medium for the cells used in the present invention. Such a medium may retain isotonicity, cell metabolism, pH, and the like. It is compatible with administration to a subject *in vivo,* and can be used, therefore, for cell delivery and treatment.

The term "potency" refers to the ability of the cells (or conditioned medium from the cells) to achieve the various effects described in this application. Accordingly, potency refers to the effect at various levels, including, but not limited to (1) reducing inflammation; (2) reducing leukocyte infiltration (neutrophils, lymphocytes, or monocytes); (3) reducing adhesion, for example, of the selectins to sialylated Lewis antigen x on leukocytes, including, but not limited to, CD4⁺ and CD8⁺ lymphocytes; and (4) reducing the expression of cellular adhesion molecules on endothelial cells, including, but not limited to, ICAM, VCAM, E-selectin, and P-selectin.

"Primordial embryonic germ cells" (PG or EG cells) can be cultured and stimulated to produce many less differentiated cell types.

"Progenitor cells" are cells produced during differentiation of a stem cell that have some, but not all, of the characteristics of their terminally-differentiated progeny. Defined progenitor cells, such as "cardiac progenitor cells," are committed to a lineage, but not to a specific or terminally differentiated cell type. The term "progenitor" as used in the acronym "MAPC" does not limit these cells to a particular lineage. A progenitor cell can form a progeny cell that is more highly differentiated than the progenitor cell.

The term "reduce" as used herein means to prevent as well as decrease. In the context of treatment, to "reduce" is to both prevent or ameliorate one or more clinical symptoms. A clinical symptom is one (or more) that has or will have, if left untreated, a negative impact on the quality of life (health) of the subject. This also applies to the biological effects such as reducing extravasation, downregulating adhesion molecules on endothelial cells, reducing adhesion of leukocytes to endothelial cells, reducing leukocyte infiltration into the surrounding tissue, reducing leukocyte binding, etc, the end result of which would be to ameliorate the deleterious effects of inflammation.

"Selecting" a cell with a desired level of potency (e.g., for reducing expression of one or more adhesion molecules) can mean identifying (as by assay), isolating, and expanding a cell. This could create a population that has a higher potency than the parent cell population from which the cell was isolated.

To select a cell would include both an assay to determine if there is the desired effect and would also include obtaining that cell. The cell may naturally have the effect in that the cell was not incubated with or exposed to an agent that induces the effect. The cell may not be known to have the effect prior to conducting the assay. As the effects could depend on gene expression and/or secretion, one could also select on the basis of one or more of the genes that cause the effects.

Selection could be from cells in a tissue. For example, in this case, cells would be isolated from a desired tissue, expanded in culture, selected for a desired effect, and the selected cells further expanded.

Selection could also be from cells ex vivo, such as cells in culture. In this case, one or more of the cells in culture would be assayed for the effect and the cells obtained that have the effect could be further expanded.

Cells could also be selected for enhanced effect. In this case, the cell population from which the enhanced cell is obtained already has the effect. Enhanced effectiveness means a higher average amount of the effect per cell than in the parent population.

The parent population from which the enhanced cell is selected may be substantially homogeneous (the same cell type). One way to obtain such an enhanced cell from this population is to create single cells or cell pools and assay those cells or cell pools for the effect to obtain clones that naturally have the effect (as opposed to treating the cells with a modulator of the effect) and then expanding those cells that are naturally enhanced.

However, cells may be treated with one or more agents that will enhance the effect of endogenous cellular pathways. Thus, substantially homogeneous populations may be treated to enhance modulation.

If the population is not substantially homogeneous, then, it is preferable that the parental cell population to be treated contains at least 100 of the effective cell type in which enhanced effect is sought, more preferably at least 1,000 of the cells, and still more preferably, at least 10,000 of the cells. Following treatment, this sub-population can be recovered from the heterogeneous population by known cell selection techniques and further expanded if desired.

Thus, desired levels of the effect may be those that are higher than the levels in a given preceding population. For example, cells that are put into primary culture from a tissue and expanded and isolated by culture conditions that are not specifically designed to have the effect, may provide a parent population. Such a parent population can be treated to enhance the average effect per cell or screened for a cell or cells within the population that express higher effect. Such cells can be expanded then to provide a population with a higher (desired) effect.

"Self-renewal" refers to the ability to produce replicate daughter stem cells having differentiation potential that is identical to those from which they arose. A similar term used in this context is "proliferation."

"Stem cell" means a cell that can undergo self-renewal (i.e., progeny with the same differentiation potential) and also produce progeny cells that are more restricted in differentiation potential. Within the context of the invention, a stem cell would also encompass a more differentiated cell that has de-differentiated, for example, by nuclear transfer, by fusion with a more primitive stem cell, by introduction of specific transcription factors, or by culture under specific conditions. See, for example, Wilmut et al., Nature, 385:810-813 (1997); Ying et al., Nature, 416:545-548 (2002); Guan et al., Nature, 440:1199-1203 (2006); Takahashi et al., Cell, 126:663-676 (2006); Okita et al., Nature, 448:313-317 (2007); and Takahashi et al., Cell, 131:861-872 (2007).

Dedifferentiation may also be caused by the administration of certain compounds or exposure to a physical environment *in vitro* or *in vivo* that would cause the dedifferentiation. Stem cells also may be derived from abnormal tissue, such as a teratocarcinoma and some other sources such as embryoid bodies (although these can be considered embryonic stem cells in that they are derived from embryonic tissue, although not directly from the inner cell mass). Stem cells may also be produced by introducing genes associated with stem cell function into a non-stem cell, such as an induced pluripotent stem cell.

"Subject" means a vertebrate, such as a mammal, such as a human. Mammals include, but are not limited to, humans, dogs, cats, horses, cows, and pigs.

The term "therapeutically effective amount" refers to the amount of an agent determined to produce any therapeutic response in a mammal. For example, effective anti-inflammatory therapeutic agents may prolong the survivability of the patient, and/or inhibit overt clinical symptoms. Treatments that are therapeutically effective within the meaning of the term as used herein, include treatments that improve a subject's quality of life even if they do not improve the disease outcome per se. Such therapeutically effective amounts are readily ascertained by one of ordinary skill in the art. Thus, to "treat" means to deliver such an amount. Thus, treating can prevent or ameliorate any pathological symptoms of inflammation.

"Treat," "treating," or "treatment" are used broadly in relation to the invention and each such term encompasses, among others, preventing, ameliorating, inhibiting, or curing a deficiency, dysfunction, disease, or other deleterious process, including those that interfere with and/or result from a therapy.

### Stem Cells

The present invention can be practiced, preferably, using stem cells of vertebrate species, such as humans, non-human primates, domestic animals, livestock, and other non-human mammals. These include, but are not limited to, those cells described below.

### Embryonic Stem Cells

The most well studied stem cell is the embryonic stem cell (ESC) as it has unlimited self-renewal and multipotent differentiation potential. These cells are derived from the inner cell mass of the blastocyst or can be derived from the primordial germ cells of a post-implantation embryo (embryonal germ cells or EG cells). ES and EG cells have been derived, first from mouse, and later, from many different animals, and more recently, also from non-human primates and humans. When introduced into mouse blastocysts or blastocysts of other animals, ESCs can contribute to all tissues of the animal. ES and EG cells can be identified by positive staining with antibodies against SSEA1 (mouse) and SSEA4 (human). See, for example, U.S. Patent Nos. 5,453,357; 5,656,479; 5,670,372; 5,843,780; 5,874,301; 5,914,268; 6,110,739 6,190,910; 6,200,806; 6,432,711; 6,436,701, 6,500,668; 6,703,279; 6,875,607; 7,029,913; 7,112,437; 7,145,057; 7,153,684; and 7,294,508, each of which is incorporated by reference for teaching embryonic stem cells and methods of making and expanding them. Accordingly, ESCs and methods for isolating and expanding them are well-known in the art.

A number of transcription factors and exogenous cytokines have been identified that influence the potency status of embryonic stem cells *in vivo.* The first transcription factor to be described that is involved in stem cell pluripotency is Oct4. Oct4 belongs to the POU (Pit-Oct-Unc) family of transcription factors and is a DNA binding protein that is able to activate the transcription of genes, containing an octameric sequence called "the octamer motif' within the promoter or enhancer region. Oct4 is expressed at the moment of the cleavage stage of the fertilized zygote until the egg cylinder is formed. The function of Oct3/4 is to repress differentiation inducing genes (i.e., FoxaD3, hCG) and to activate genes promoting pluripotency (FGF4, Utf1, Rex1). Sox2, a member of the high mobility group (HMG) box transcription factors, cooperates with Oct4 to activate transcription of genes expressed in the inner cell mass. It is essential that Oct3/4 expression in embryonic stem cells is maintained between certain levels. Overexpression or downregulation of >50% of Oct4 expression level will alter embryonic stem cell fate, with the formation of primitive endoderm/mesoderm or trophectoderm, respectively. *In vivo,* Oct4 deficient embryos develop to the blastocyst stage, but the inner cell mass cells are not pluripotent. Instead they differentiate along the extraembryonic trophoblast lineage. Sall4, a mammalian Spalt transcription factor, is an upstream regulator of Oct4, and is therefore important to maintain appropriate levels of Oct4 during early phases of embryology. When Sall4 levels fall below a certain threshold, trophectodermal cells will expand ectopically into the inner cell mass. Another transcription factor required for pluripotency is Nanog, named after a Celtic tribe "Tir Nan Og": the land of the ever young. *In vivo,* Nanog is expressed from the stage of the compacted morula, is subsequently defined to the inner cell mass and is downregulated by the implantation stage. Downregulation of Nanog may be important to avoid an uncontrolled expansion of pluripotent cells and to allow multilineage differentiation during gastrulation. Nanog null embryos, isolated at day 5.5, consist of a disorganized blastocyst, mainly containing extraembryonic endoderm and no discernable epiblast.

### Non-Embryonic Stem Cells

Stem cells have been identified in most tissues. Perhaps the best characterized is the hematopoietic stem cell (HSC). HSCs are mesoderm-derived cells that can be purified using cell surface markers and functional characteristics. They have been isolated from bone marrow, peripheral blood, cord blood, fetal liver, and yolk sac. They initiate hematopoiesis and generate multiple hematopoietic lineages. When transplanted into lethally-irradiated animals, they can repopulate the erythroid neutrophil-macrophage, megakaryocyte, and lymphoid hematopoietic cell pool. They can also be induced to undergo some self-renewal cell division. See, for example, U.S. Patent Nos. 5,635,387; 5,460,964; 5,677,136; 5,750,397; 5,681,599; and 5,716,827. U.S. Patent No. 5,192,553 reports methods for isolating human neonatal or fetal hematopoietic stem or progenitor cells. U.S. Patent No. 5,716,827 reports human hematopoietic cells that are Thy-1⁺ progenitors, and appropriate growth media to regenerate them *in vitro.* U.S. Patent No. 5,635,387 reports a method and device for culturing human hematopoietic cells and their precursors. U.S. Patent No. 6,015,554 describes a method of reconstituting human lymphoid and dendritic cells. Accordingly, HSCs and methods for isolating and expanding them are well-known in the art.

Another stem cell that is well-known in the art is the neural stem cell (NSC). These cells can proliferate *in vivo* and continuously regenerate at least some neuronal cells. When cultured ex vivo, neural stem cells can be induced to proliferate as well as differentiate into different types of neurons and glial cells. When transplanted into the brain, neural stem cells can engraft and generate neural and glial cells. See, for example, Gage F.H., Science, 287:1433-1438 (2000), Svendsen S.N. et al, Brain Pathology, 9:499-513 (1999), and Okabe S. et al., Mech Development, 59:89-102 (1996). U.S. Patent No. 5,851,832 reports multipotent neural stem cells obtained from brain tissue. U.S. Patent No. 5,766,948 reports producing neuroblasts from newborn cerebral hemispheres. U.S. Patent Nos. 5,564,183 and 5,849,553 report the use of mammalian neural crest stem cells. U.S. Patent No. 6,040,180 reports *in vitro* generation of differentiated neurons from cultures of mammalian multipotential CNS stem cells. WO 98/50526 and WO 99/01159 report generation and isolation of neuroepithelial stem cells, oligodendrocyte-astrocyte precursors, and lineage-restricted neuronal precursors. U.S. Patent No. 5,968,829 reports neural stem cells obtained from embryonic forebrain. Accordingly, neural stem cells and methods for making and expanding them are well-known in the art.

Another stem cell that has been studied extensively in the art is the mesenchymal stem cell (MSC). MSCs are derived from the embryonal mesoderm and can be isolated from many sources, including adult bone marrow, peripheral blood, fat, placenta, and umbilical blood, among others. MSCs can differentiate into many mesodermal tissues, including muscle, bone, cartilage, fat, and tendon. There is considerable literature on these cells. See, for example, U.S. Patent Nos. 5,486,389; 5,827,735; 5,811,094; 5,736,396; 5,837,539; 5,837,670; and 5,827,740. See also Pittenger, M. et al, Science, 284:143-147 (1999).

Another example of an adult stem cell is adipose-derived adult stem cells (ADSCs) which have been isolated from fat, typically by liposuction followed by release of the ADSCs using collagenase. ADSCs are similar in many ways to MSCs derived from bone marrow, except that it is possible to isolate many more cells from fat. These cells have been reported to differentiate into bone, fat, muscle, cartilage, and neurons. A method of isolation has been described in U.S. 2005/0153442.

Other stem cells that are known in the art include gastrointestinal stem cells, epidermal stem cells, and hepatic stem cells, which have also been termed "oval cells" (Potten, C., et al., Trans R Soc Lond B Biol Sci, 353:821-830 (1998), Watt, F., Trans R Soc Lond B Biol Sci, 353:831 (1997); Alison et al., Hepatology, 29:678-683 (1998).

Other non-embryonic cells reported to be capable of differentiating into cell types of more than one embryonic germ layer include, but are not limited to, cells from umbilical cord blood (see U.S. Publication No. 2002/0164794), placenta (see U.S. Publication No. 2003/0181269, umbilical cord matrix (Mitchell, K.E. et al., Stem Cells, 21:50-60 (2003)), small embryonic-like stem cells (Kucia, M. et al., J Physiol Pharmacol, 57 Suppl 5:5-18 (2006)), amniotic fluid stem cells (Atala, A., J Tissue Regen Med, 1:83-96 (2007)), skin-derived precursors (Toma et al., Nat Cell Biol, 3:778-784 (2001)), and bone marrow (see U.S. Publication Nos. 2003/0059414 and 2006/0147246), each of which is incorporated by reference for teaching these cells.

### Strategies of Reprogramming Somatic Cells

Several different strategies such as nuclear transplantation, cellular fusion, and culture induced reprogramming have been employed to induce the conversion of differentiated cells into an embryonic state. Nuclear transfer involves the injection of a somatic nucleus into an enucleated oocyte, which, upon transfer into a surrogate mother, can give rise to a clone ("reproductive cloning"), or, upon explantation in culture, can give rise to genetically matched embryonic stem (ES) cells ("somatic cell nuclear transfer," SCNT). Cell fusion of somatic cells with ES cells results in the generation of hybrids that show all features of pluripotent ES cells. Explantation of somatic cells in culture selects for immortal cell lines that may be pluripotent or multipotent. At present, spermatogonial stem cells are the only source of pluripotent cells that can be derived from postnatal animals. Transduction of somatic cells with defined factors can initiate reprogramming to a pluripotent state. These experimental approaches have been extensively reviewed (Hochedlinger and Jaenisch, Nature, 441:1061-1067 (2006) and Yamanaka, S., Cell Stem Cell, 1:39-49 (2007)).

### Nuclear Transfer

Nuclear transplantation (NT), also referred to as somatic cell nuclear transfer (SCNT), denotes the introduction of a nucleus from a donor somatic cell into an enucleated ogocyte to generate a cloned animal such as Dolly the sheep (Wilmut et al., Nature, 385:810-813 (1997). The generation of live animals by NT demonstrated that the epigenetic state of somatic cells, including that of terminally differentiated cells, while stable, is not irreversible fixed but can be reprogrammed to an embryonic state that is capable of directing development of a new organism. In addition to providing an exciting experimental approach for elucidating the basic epigenetic mechanisms involved in embryonic development and disease, nuclear cloning technology is of potential interest for patient-specific transplantation medicine.

### Fusion of Somatic Cells and Embryonic Stem Cells

Epigenetic reprogramming of somatic nuclei to an undifferentiated state has been demonstrated in murine hybrids produced by fusion of embryonic cells with somatic cells. Hybrids between various somatic cells and embryonic carcinoma cells (Solter, D., Nat Rev Genet, 7:319-327 (2006), embryonic germ (EG), or ES cells (Zwaka and Thomson, Development, 132:227-233 (2005)) share many features with the parental embryonic cells, indicating that the pluripotent phenotype is dominant in such fusion products. As with mouse (Tada et al., Curr Biol, 11:1553-1558 (2001)), human ES cells have the potential to reprogram somatic nuclei after fusion (Cowan et al., Science, 309:1369-1373(2005)); Yu et al., Science, 318:1917-1920 (2006)). Activation of silent pluripotency markers such as Oct4 or reactivation of the inactive somatic X chromosome provided molecular evidence for reprogramming of the somatic genome in the hybrid cells. It has been suggested that DNA replication is essential for the activation of pluripotency markers, which is first observed 2 days after fusion (Do and Scholer, Stem Cells, 22:941-949 (2004)), and that forced overexpression of Nanog in ES cells promotes pluripotency when fused with neural stem cells (Silva et al., Nature, 441:997-1001 (2006)).

### Culture-Induced Reprogramming

Pluripotent cells have been derived from embryonic sources such as blastomeres and the inner cell mass (ICM) of the blastocyst (ES cells), the epiblast (EpiSC cells), primordial germ cells (EG cells), and postnatal spermatogonial stem cells ("maGSCsm" "ES-like" cells). The following pluripotent cells, along with their donor cell/tissue is as follows: parthogenetic ES cells are derived from murine oocytes (Narasimha et al., Curr Biol, 7:881-884 (1997)); embryonic stem cells have been derived from blastomeres (Wakayama et al., Stem Cells, 25:986-993 (2007)); inner cell mass cells (source not applicable) (Eggan et al., Nature, 428:44-49 (2004)); embryonic germ and embryonal carcinoma cells have been derived from primordial germ cells (Matsui et al., Cell, 70:841-847 (1992)); GMCS, maSSC, and MASC have been derived from spermatogonial stem cells (Guan et al., Nature, 440:1199-1203 (2006); Kanatsu-Shinohara et al., Cell, 119:1001-1012 (2004); and Seandel et al., Nature, 449:346-350 (2007)); EpiSC cells are derived from epiblasts (Brons et al., Nature, 448:191-195 (2007); Tesar et al., Nature, 448:196-199(2007)); parthogenetic ES cells have been derived from human oocytes (Cibelli et al., Science, 295L819 (2002); Revazova et al., Cloning Stem Cells, 9:432-449 (2007)); human ES cells have been derived from human blastocysts (Thomson et al., Science, 282:1145-1147 (1998)); MAPC have been derived from bone marrow (Jiang et al., Nature, 418:41-49 (2002); Phinney and Prockop, Stem Cells, 25:2896-2902 (2007)); cord blood cells (derived from cord blood) (van de Ven et al., Exp Hematol, 35:1753-1765 (2007)); neurosphere derived cells derived from neural cell (Clarke et al., Science, 288:1660-1663 (2000)). Donor cells from the germ cell lineage such as PGCs or spermatogonial stem cells are known to be unipotent *in vivo,* but it has been shown that pluripotent ES-like cells (Kanatsu-Shinohara et al., Cell, 119:1001-1012 (2004) or maGSCs (Guan et al., Nature, 440:1199-1203 (2006), can be isolated after prolonged *in vitro* culture. While most of these pluripotent cell types were capable of in vitro differentiation and teratoma formation, only ES, EG, EC, and the spermatogonial stem cell-derived maGCSs or ES-like cells were pluripotent by more stringent criteria, as they were able to form postnatal chimeras and contribute to the germ line. Recently, multipotent adult spermatogonial stem cells (MASCs) were derived from testicular spermatogonial stem cells of adult mice, and these cells had an expression profile different from that of ES cells (Seandel et al., Nature, 449:346-350 (2007)) but similar to EpiSC cells, which were derived from the epiblast of post-implantation mouse embryos (Brons et al., Nature, 448:191-195 (2007); Tesar et al., Nature, 448:196-199 (2007)).

### Reprogramming by Defined Transcription Factors

Takahashi and Yamanaka have reported reprogramming somatic cells back to an ES-like state (Takahashi and Yamanaka, Cell, 126:663-676 (2006)). They successfully reprogrammed mouse embryonic fibroblasts (MEFs) and adult fibroblasts to pluripotent ES-like cells after viral-mediated transduction of the four transcription factors Oct4, Sox2, c-myc, and Klf4 followed by selection for activation of the Oct4 target gene Fbx15 (Figure 2A). Cells that had activated Fbx15 were coined iPS (induced pluripotent stem) cells and were shown to be pluripotent by their ability to form teratomas, although the were unable to generate live chimeras. This pluripotent state was dependent on the continuous viral expression of the transduced Oct4 and Sox2 genes, whereas the endogenous Oct4 and Nanog genes were either not expressed or were expressed at a lower level than in ES cells, and their respective promoters were found to be largely methylated. This is consistent with the conclusion that the Fbx15-iPS cells did not correspond to ES cells but may have represented an incomplete state of reprogramming. While genetic experiments had established that Oct4 and Sox2 are essential for pluripotency (Chambers and Smith, Oncogene, 23:7150-7160 (2004); Ivanona et al., Nature, 442:5330538 (2006); Masui et al., Nat Cell Biol, 9:625-635 (2007)), the role of the two oncogenes c-myc and Klf4 in reprogramming is less clear. Some of these oncogenes may, in fact, be dispensable for reprogramming, as both mouse and human iPS cells have been obtained in the absence of c-myc transduction, although with low efficiency (Nakagawa et al., Nat Biotechnol, 26:191-106 (2008); Werning et al., Nature, 448:318-324 (2008); Yu et al., Science, 318: 1917-1920 (2007)).

### MAPC

MAPC is an acronym for "multipotent adult progenitor cell" (non-ES, non-EG, non-germ). MAPC have the capacity to differentiate into cell types of at least two, such as, all three, primitive germ layers (ectoderm, mesoderm, and endoderm). Genes found in ES cells may also be found in MAPC (e.g., telomerase, Oct 3/4, rex-1, rox-1, sox-2). Oct 3/4 (Oct 3A in humans) appears to be specific for ES and germ cells. MAPC represents a more primitive progenitor cell population than MSC (Verfaillie, C.M., Trends Cell Biol 12:502-8 (2002), Jahagirdar, B.N., et al., Exp Hematol, 29:543-56 (2001); Reyes, M. and C.M. Verfaillie, Ann N Y Acad Sci, 938:231-233 (2001); Jiang, Y. et al., Exp Hematol, 30896-904 (2002); and (Jiang, Y. et al., Nature, 418:41-9. (2002)).

Human MAPCs are described in U.S. Patent 7,015,037 and U.S. Application No. 10/467,963. MAPCs have been identified in other mammals. Murine MAPCs, for example, are also described in U.S. Patent 7,015,037 and U.S. Application No. 10/467,963. Rat MAPCs are also described in U.S. Application No. 10/467,963.

These references are incorporated by reference for describing MAPCs first isolated by Catherine Verfaillie.

### Isolation and Growth of MAPCs

Methods of MAPC isolation are known in the art. See, for example, U.S. Patent 7,015,037 and U.S. Application No. 10/467,963, and these methods, along with the characterization (phenotype) of MAPCs, are incorporated herein by reference. MAPCs can be isolated from multiple sources, including, but not limited to, bone marrow, placenta, umbilical cord and cord blood, muscle, brain, liver, spinal cord, blood or skin. It is, therefore, possible to obtain bone marrow aspirates, brain or liver biopsies, and other organs, and isolate the cells using positive or negative selection techniques available to those of skill in the art, relying upon the genes that are expressed (or not expressed) in these cells (e.g., by functional or morphological assays such as those disclosed in the above-referenced applications, which have been incorporated herein by reference).

### MAPCs from Human Bone Marrow as Described in U.S. Patent 7,015,037

MAPCs do not express the common leukocyte antigen CD45 or erythroblast specific glycophorin-A (Gly-A). The mixed population of cells was subjected to a Ficoll Hypaque separation. The cells were then subjected to negative selection using anti-CD45 and anti-Gly-A antibodies, depleting the population of CD45⁺ and Gly-A⁺ cells, and the remaining approximately 0.1% of marrow mononuclear cells were then recovered. Cells could also be plated in fibronectin-coated wells and cultured as described below for 2-4 weeks to deplete the cells of CD45⁺ and Gly-A⁺ cells. In cultures of adherent bone marrow cells, many adherent stromal cells undergo replicative senescence around cell doubling 30 and a more homogenous population of cells continues to expand and maintains long telomeres.

Alternatively, positive selection could be used to isolate cells via a combination of cell-specific markers. Both positive and negative selection techniques are available to those of skill in the art, and numerous monoclonal and polyclonal antibodies suitable for negative selection purposes are also available in the art (see, for example, Leukocyte Typing V, Schlossman, et al., Eds. (1995) Oxford University Press) and are commercially available from a number of sources.

Techniques for mammalian cell separation from a mixture of cell populations have also been described by Schwartz, et al., in U. S. Patent No. 5,759,793 (magnetic separation), Basch et al., 1983 (immunoaffinity chromatography), and Wysocki and Sato, 1978 (fluorescence-activated cell sorting).

### Culturing MAPCs as Described in U.S. 7,015,037

MAPCs isolated as described herein can be cultured using methods disclosed herein and in U.S. Patent 7,015,037, which is incorporated by reference for these methods.

Cells may be cultured in low-serum or serum-free culture medium. Serum-free medium used to culture MAPCs is described in U.S. Patent 7,015,037. Many cells have been grown in serum-free or low-serum medium. In this case, the medium is supplemented with one or more growth factors. Commonly-used growth factors include but are not limited to bone morphogenic protein, basis fibroblast growth factor, platelet-derived growth factor, and epidermal growth factor. See, for example, U.S. Patent Nos. 7,169,610; 7,109,032; 7,037,721; 6,617,161; 6,617,159; 6,372,210;6,224,860; 6,037,174; 5,908,782; 5,766,951; 5,397,706; and 4,657,866; all incorporated by reference for teaching growing cells in serum-free medium.

### Additional Culture Methods

In additional experiments the density at which MAPCs are cultured can vary from about 100 cells/cm² or about 150 cells/cm² to about 10,000 cells/cm², including about 200 cells/cm² to about 1500 cells/cm² to about 2000 cells/cm². The density can vary between species. Additionally, optimal density can vary depending on culture conditions and source of cells. It is within the skill of the ordinary artisan to determine the optimal density for a given set of culture conditions and cells.

Also, effective atmospheric oxygen concentrations of less than about 10%, including about 1-5% and, especially, 3-5%, can be used at any time during the isolation, growth and differentiation of MAPCs in culture.

Cells may be cultured under various serum concentrations, e.g., about 2-20%. Fetal bovine serum may be used. Higher serum may be used in combination with lower oxygen tensions, for example, about 15-20%. Cells need not be selected prior to adherence to culture dishes. For example, after a Ficoll gradient, cells can be directly plated, e.g., 250,000-500,000/cm². Adherent colonies can be picked, possibly pooled, and expanded.

In one embodiment, used in the experimental procedures in the Examples, high serum (around 15-20%) and low oxygen (around 3-5%) conditions were used for the cell culture. Specifically, adherent cells from colonies were plated and passaged at densities of about 1700-2300 cells/cm² in 18% serum and 3% oxygen (with PDGF and EGF).

In an embodiment specific for MAPCs, supplements are cellular factors or components that allow MAPCs to retain the ability to differentiate into all three lineages. This may be indicated by the expression of specific markers of the undifferentiated state. MAPCs, for example, constitutively express Oct 3/4 (Oct 3A) and maintain high levels of telomerase.

### Cell Culture

For all the components listed below, see U.S. 7,015,037, which is incorporated by reference for teaching these components.

In general, cells useful for the invention can be maintained and expanded in culture medium that is available and well-known in the art. Also contemplated is supplementation of cell culture medium with mammalian sera. Additional supplements can also be used advantageously to supply the cells with the necessary trace elements for optimal growth and expansion. Hormones can also be advantageously used in cell culture. Lipids and lipid carriers can also be used to supplement cell culture media, depending on the type of cell and the fate of the differentiated cell. Also contemplated is the use of feeder cell layers.

Cells may also be grown in "3D" (aggregated) cultures. An example is PCT/US2009/31528, filed January 21, 2009.

Once established in culture, cells can be used fresh or frozen and stored as frozen stocks, using, for example, DMEM with 40% FCS and 10% DMSO. Other methods for preparing frozen stocks for cultured cells are also available to those of skill in the art.

### Pharmaceutical Formulations

U.S. 7,015,037 is incorporated by reference for teaching pharmaceutical formulations. In certain embodiments, the cell populations are present within a composition adapted for and suitable for delivery, i.e., physiologically compatible.

In some embodiments the purity of the cells (or conditioned medium) for administration to a subject is about 100% (substantially homogeneous). In other embodiments it is 95% to 100%. In some embodiments it is 85% to 95%. Particularly, in the case of admixtures with other cells, the percentage can be about 10%-15%, 15%-20%, 20%-25%, 25%-30%, 30%-35%, 35%-40%, 40%-45%, 45%-50%, 60%-70%, 70%-80%, 80%-90%, or 90%-95%. Or isolation/purity can be expressed in terms of cell doublings where the cells have undergone, for example, 10-20, 20-30, 30-40, 40-50 or more cell doublings.

The choice of formulation for administering the cells for a given application will depend on a variety of factors. Prominent among these will be the species of subject, the nature of the condition being treated, its state and distribution in the subject, the nature of other therapies and agents that are being administered, the optimum route for administration, survivability via the route, the dosing regimen, and other factors that will be apparent to those skilled in the art. For instance, the choice of suitable carriers and other additives will depend on the exact route of administration and the nature of the particular dosage form.

Final formulations of the aqueous suspension of cells/medium will typically involve adjusting the ionic strength of the suspension to isotonicity (i.e., about 0.1 to 0.2) and to physiological pH (i.e., about pH 6.8 to 7.5). The final formulation will also typically contain a fluid lubricant.

In some embodiments, cells/medium are formulated in a unit dosage injectable form, such as a solution, suspension, or emulsion. Pharmaceutical formulations suitable for injection of cells/medium typically are sterile aqueous solutions and dispersions. Carriers for injectable formulations can be a solvent or dispersing medium containing, for example, water, saline, phosphate buffered saline, polyol (for example, glycerol, propylene glycol, liquid polyethylene glycol, and the like), and suitable mixtures thereof.

The skilled artisan can readily determine the amount of cells and optional additives, vehicles, and/or carrier in compositions to be administered in methods of the invention. Typically, any additives (in addition to the cells) are present in an amount of 0.001 to 50 wt % in solution, such as in phosphate buffered saline. The active ingredient is present in the order of micrograms to milligrams, such as about 0.0001 to about 5 wt %, preferably about 0.0001 to about 1 wt %, most preferably about 0.0001 to about 0.05 wt % or about 0.001 to about 20 wt %, preferably about 0.01 to about 10 wt %, and most preferably about 0.05 to about 5 wt %.

The dosage of the cells will vary within wide limits and will be fitted to the individual requirements in each particular case. In general, in the case of parenteral administration, it is customary to administer from about 0.01 to about 20 million cells/kg of recipient body weight. The number of cells will vary depending on the weight and condition of the recipient, the number or frequency of administrations, and other variables known to those of skill in the art. The cells can be administered by a route that is suitable for the tissue or organ. For example, they can be administered systemically, i.e., parenterally, by intravenous administration, or can be targeted to a particular tissue or organ; they can be administrated via subcutaneous administration or by administration into specific desired tissues.

The cells can be suspended in an appropriate excipient in a concentration from about 0.01 to about 5x10⁶ cells/ml. Suitable excipients for injection solutions are those that are biologically and physiologically compatible with the cells and with the recipient, such as buffered saline solution or other suitable excipients. The composition for administration can be formulated, produced, and stored according to standard methods complying with proper sterility and stability.

### Administration into Lymphohematopoietic Tissues

Techniques for administration into these tissues are known in the art. For example, intra-bone marrow injections can involve injecting cells directly into the bone marrow cavity typically of the posterior iliac crest but may include other sites in the iliac crest, femur, tibia, humerus, or ulna; splenic injections could involve radiographic guided injections into the spleen or surgical exposure of the spleen via laparoscopic or laparotomy; Peyer's patches, GALT, or BALT injections could require laparotomy or laparoscopic injection procedures.

### Dosing

Doses for humans or other mammals can be determined without undue experimentation by the skilled artisan, from this disclosure, the documents cited herein, and the knowledge in the art. The dose of cells/medium appropriate to be used in accordance with various embodiments of the invention will depend on numerous factors. The parameters that will determine optimal doses to be administered for primary and adjunctive therapy generally will include some or all of the following: the disease being treated and its stage; the species of the subject, their health, gender, age, weight, and metabolic rate; the subject's immunocompetence; other therapies being administered; and expected potential complications from the subject's history or genotype. The parameters may also include: whether the cells are syngeneic, autologous, allogeneic, or xenogeneic; their potency (specific activity); the site and/or distribution that must be targeted for the cells/medium to be effective; and such characteristics of the site such as accessibility to cells/medium and/or engraftment of cells. Additional parameters include coadministration with other factors (such as growth factors and cytokines). The optimal dose in a given situation also will take into consideration the way in which the cells/medium are formulated, the way they are administered, and the degree to which the cells/medium will be localized at the target sites following administration.

The optimal dose of cells could be in the range of doses used for autologous, mononuclear bone marrow transplantation. For fairly pure preparations of cells, optimal doses in various embodiments will range from 10⁴ to 10⁸ cells/kg of recipient mass per administration. In some embodiments the optimal dose per administration will be between 10⁵ to 10⁷ cells/kg. In many embodiments the optimal dose per administration will be 5 x 10⁵ to 5 x 10⁶ cells/kg. By way of reference, higher doses in the foregoing are analogous to the doses of nucleated cells used in autologous mononuclear bone marrow transplantation. Some of the lower doses are analogous to the number of CD34⁺ cells/kg used in autologous mononuclear bone marrow transplantation.

In various embodiments, cells/medium may be administered in an initial dose, and thereafter maintained by further administration. Cells/medium may be administered by one method initially, and thereafter administered by the same method or one or more different methods. The levels can be maintained by the ongoing administration of the cells/medium. Various embodiments administer the cells/medium either initially or to maintain their level in the subject or both by intravenous injection. In a variety of embodiments, other forms of administration, are used, dependent upon the patient's condition and other factors, discussed elsewhere herein.

Cells/medium may be administered in many frequencies over a wide range of times. Generally lengths of treatment will be proportional to the length of the disease process, the effectiveness of the therapies being applied, and the condition and response of the subject being treated.

### Uses

Because the cells of the invention secrete one or more factors that ultimately reduce inflammation through the various biological mechanisms described in this application, administering the cells is useful to reduce undesirable inflammation in any number of pathologies, as listed above

In addition, other uses are provided by knowledge of the biological mechanisms described in this application. One of these includes drug discovery. This aspect involves screening one or more compounds for the ability to modulate the anti-inflammatory effects of the cells. This would involve, first, developing an assay for the cell's ability to reduce any of the following: (1) inflammation, (2) extravasation, (3) endothelial cell-leukocyte binding, (4) expression of adhesion molecules in endothelial cells (RNA and/or protein), (5) expression of the cognate ligand (located on a leukocyte) that binds the adhesion cell molecule, if expression of this ligand is modulated by the endothelial cell, and (6) production of cytokines by endothelial cells. Accordingly, the assay may be designed to be conducted in vivo or in vitro. Modulation assays could assess the activation state at any desired level, e.g., morphological, gene expression, functional, etc. It may involve isolated vascular endothelium. Alternatively, it may involve vascular endothelial cells partly or wholly removed from endothelium, including endothelial cell strains and endothelial cell lines, both natural and recombinant. However, it may also include isolated cellular components known to have binding affinity such as adhesion molecules that are expressed on the endothelial cells and their cognate binding ligands that are found on leukocytes. Thus, recombinant cells expressing or secreting the cellular adhesion molecule and the cognate leukocyte ligand could be used to assay binding. Or the isolated lymphocyte binding partner could be used with the cell expressing the endothelial adhesion molecule, such as P-selectin or E-selectin and CD15s; ICAM-1 or ICAM-2 and LFA-1; ICAM and Mac-1; VCAM-1 and VLA-4.

The assay may contain an activating cytokine, such as TNF-α, or IL-1β. The cytokine alone may form the basis for the assay. For example, the cells/medium could be assayed for the ability to bind TNF-α or act as competitive inhibitor of TNF-α receptor in a cell-based receptor assay or with soluble receptor. Unbound TNF-α could be detected directly or the assay for the unbound TNF-α could involve an assay for any of the biological effects of TNF-α. This applies also to any of the other activating cytokines, e.g., IL-1.

Or the assay could involve the ability of the cell/medium to modulate (increase, decrease) NFκB in a receptor-based assay with a reporter gene operably linked to a promoter controlled by NFκB. The assay would then be used to screen for compounds/agents that increase or decrease the effect of the cells/medium.

Gene expression can be assessed by directly assaying protein or RNA. This can be done through any of the well-known techniques available in the art, such as by FACS and other antibody-based detection methods and PCR and other hybridization-based detection methods. Indirect assays may also be used for expression, such as binding to any of the known binding partners.

Assays for expression/secretion include, but are not limited to, ELISA, Luminex. qRT-PCR, anti-factor western blots, and factor immunohistochemistry on tissue samples or cells.

Quantitative determination of modulatory factors in cells and conditioned media can be performed using commercially available assay kits (e.g., R&D Systems that relies on a two-step subtractive antibody-based assay).

The invention encompasses methods to produce cells with increased potency as described herein. Accordingly, the invention encompasses methods to identify compounds that increase the ability of the cell to have any of the effects described herein by exposing the cells to a compound and assaying for the ability of the cells to achieve the effect at any desired level. In one embodiment, some cells may require contact with activated endothelial cells before producing the factors that have the effects described herein. These cells may be designated "naive." Accordingly, in one embodiment, it is possible to substitute for the effect of activated endothelial cells by the use of the compounds that pertain to drug discovery described in this application. In one embodiment, for example, the inventors have found that a combination of IL-1β, TNF-α, and IFN-γ can substitute for contact with activated endothelial cells as briefly described in the exemplary section. Accordingly, cells may be screened with any number of compounds to identify compounds that allow this substitution. These compounds can then be used to pre-treat naive cells for any of the therapeutic uses described in this application, as well as producing compositions that are useful for clinical, therapeutic, and diagnostic applications and cell banking.

### Examples of Specific Assays

1. FACS analysis of endothelial cells to examine the presence of adhesion molecules after co-culture with MAPCs or incubated with conditioned media from MAPCs.
2. Quantitation of neutrophil binding to endothelial cells co-culture with MAPCs or incubated with conditioned media from MAPCs.
3. Quantitation of NF-kB activity using a promoter-based system after co-culture with MAPCs or incubated with conditioned media from MAPCs.
4. Quantation of leukocyte extravasation through an endothelial layer using an ECIS based system or similar system, after the endothelial layer has been co-cultured with MAPCs or incubated with conditioned media from MAPCs.

Assays for potency may also be performed by detecting the active factors secreted by the cells. These may include glucocorticoids, HB-EGF, IL-10, Prostaglandin A1, IL-13, IL-1R, IL-18R, IFN-R, TNF-R1, TNF-R2, IL-4, IL-11, IFN-β, TGF-β1, β2, β3, Prostaglandin E2, SPP1, CYLD, Elastase, VEGF, IL-33, thymosin B4, and TGF- β adrenomedullin. Detection may be direct, e.g., via RNA or protein assays or indirect, e.g., biological assays for one or more biological effects of these factors.

A further use for the invention is the establishment of cell banks to provide cells for clinical administration. Generally, a fundamental part of this procedure is to provide cells that have a desired potency for administration in various therapeutic clinical settings.

Any of the same assays useful for drug discovery could also be applied to selecting cells for the bank as well as from the bank for administration.

Accordingly, in a banking procedure, the cells (or medium) would be assayed for the ability to achieve any of the above effects. Then, cells would be selected that have a desired potency for any of the above effects, and these cells would form the basis for creating a cell bank.

It is also contemplated that potency can be increased by treatment with an exogenous compound, such as a compound discovered through screening the cells with large combinatorial libraries. These compound libraries may be libraries of agents that include, but are not limited to, small organic molecules, antisense nucleic acids, siRNA DNA aptamers, peptides, antibodies, non-antibody proteins, cytokines, chemokines, and chemo-attractants. For example, cells may be exposed such agents at any time during the growth and manufacturing procedure. The only requirement is that there be sufficient numbers for the desired assay to be conducted to assess whether or not the agent increases potency. Such an agent, found during the general drug discovery process described above, could more advantageously be applied during the last passage prior to banking.

Cells are isolated from a qualified marrow donor that has undergone specific testing requirements to determine that a cell product that is obtained from this donor would be safe to be used in a clinical setting. The mononuclear cells are isolated using either a manual or automated procedure. These mononuclear cells are placed in culture allowing the cells to adhere to the treated surface of a cell culture vessel. The MAPC cells are allowed to expand on the treated surface with media changes occurring on day 2 and day 4. On day 6, the cells are removed from the treated substrate by either mechanical or enzymatic means and replated onto another treated surface of a cell culture vessel. On days 8 and 10, the cells are removed from the treated surface as before and replated. On day 13, the cells are removed from the treated surface, washed and combined with a cryoprotectant material and frozen, ultimately, in liquid nitrogen. After the cells have been frozen for at least one week, an aliquot of the cells is removed and tested for potency, identity, sterility and other tests to determine the usefulness of the cell bank. These cells in this bank can then be used by thawing them, placing them in culture or use them out of the freeze to treat potential indications.

Another use is a diagnostic assay for efficiency and beneficial clinical effect following administration of the cells. Depending on the indication, there may be biomarkers available to assess. For example, high levels of C-reactive protein are associated with acute inflammatory response. One could monitor the levels of CRP to determine beneficial clinical effects.

A further use is to assess the efficacy of the cell to achieve any of the above results as a pre-treatment diagnostic that precedes administering the cells to a subject.

### Compositions

The invention is also directed to cell populations with specific potencies for achieving any of the effects described herein (i.e., inflammation, extravasation, adhesion, reducing endothelial activation, etc.). As described above, these populations are established by selecting for cells that have desired potency. These populations are used to make other compositions, for example, a cell bank comprising populations with specific desired potencies and pharmaceutical compositions containing a cell population with a specific desired potency.

### EXAMPLES

### Example 1

MultiStem® is the trademarked designation for the MAPC cell preparation used in the experimental procedures described in this Example.

### Multipotent Adult Progenitor Cells Modulates Endothelial Cell Adhesion Molecule Surface Expression Following Activation and Reduces Inflammation Following AMI

### Rationale/Background

Localization of immune cells to a site of inflammation is an important part of the immune response to injury and infection. In response to inflammation, immune cells including leukocytes, lymphocytes and monocytes bind to, and transmigrate across, the endothelial cell layer to the site of injury^{1, 2}. Endothelial cells can be activated by thrombin, histamines, pro-inflammatory cytokines (e.g. TNF-α, Interleukin 1β) and oxygen radicals which leads to the upregulation of adhesion molecules including E-selectins, V-CAM and I-CAM-1³⁻⁵. Upregulation of cell adhesion molecules on the cell surface of endothelial cells allow activated immune cells to adhere, role and transmigrate across the endothelial cell barrier⁴.

Although mobilization of the immune system is a critical part of the immune response to infection and to wound healing, inflammation following ischemic injury can contribute to cell toxicity and death. For example, following acute myocardial infarction, an increase in neutrophil infiltration is associated with an increased risk of adverse events in patients treated with thrombolytic therapy and primary angioplasty⁶⁻⁸. The extent of the inflammatory response to myocardial infarction has a significant role in determining infarct size and subsequent left ventricular (LV) remodeling^{9, 10}. Localization of neutrophils to the heart contributes to myocardial injury by releasing proteases and reactive oxygen species (ROS) which contribute to left ventricular remodeling. Additionally, neutrophils contribute to microvascular and capillary injury by blocking capillaries in a phenomenon described as "no reflow"¹¹. A proinflammatory imbalance towards T helper 1 (Th1) cells contributes to left ventricular dilation, systolic dysfunction and reduced function¹². Therefore, modulation of the neutrophil and inflammatory cell response following AMI would help to reduce injury to the myocardium.

MultiStem® are expanded human clinical grade allogeneic multipotent adult progenitor cells that have been shown to improve cardiac function following AMI in pre-clinical models and are now in Phase I clinical trials¹³⁻¹⁵. Delivery of MultiStem into peri-infarct sites following induction of myocardial infarction by direct left anterior descending ligation results in improved cardiac function in animal models. Compared to vehicle controls, MultiStem treated animals in these studies showed improved left ventricular contractile performance, decreased scar area, increased vascular density and improved myocardial energetic characterisfics^{14,16,17}. Due to low levels of engraftment of MultiStem and minimal differentiation of MultiStem into myocardium or endothelial cells, the benefits of MultiStem during AMI are believed to be derived from paracrine effects.

Previous studies have documented that rodent and human MultiStem display potent immunosuppressive properties, and demonstrated that MultiStem cultures are non-immunogenic *in-vitro* and capable of suppressing antibody activated T-cell proliferation^{13, 15}. It was hypothesized that MultiStem's immunosuppressive or anti-inflammatory properties extend to endothelial cells and that MultiStem may confer benefit during AMI, in part, through immunomodulation of endothelial cell activation and immune cell adhesion that results in decreased neutrophil infiltration.

In this study, MultiStem was examined to determine whether it can modulate endothelial cell activation by examining the upregulation of cell adhesion molecules to the cell surface. Co-culture of MultiStem with aortic or pulmonary endothelial cells was found to prevent upregulation of E-selectin, V-CAM and to a lesser degree, I-CAM, to the cell surface of endothelial cells upon activation with TNF-α. Upregulation of E-selectin does not appear to be a result of increased cleavage from the cell surface. Instead, MultiStem modulates cell surface upregulation through decreasing transcription of V-CAM, I-CAM, and E-selectin. The reduction in cell surface adhesion molecule expression observed upon co-culture with MultiStem results in decreased neutrophil binding to endothelial cells as compared with untreated controls. This activity is not common to all bone marrow derived adherent stem cells since MSC are unable to modulate V-CAM, E-selectin or I-CAM cell surface upregulation upon activation with TNF-α. These results suggest MSC and MultiStem have distinct secretion profiles in response to inflammatory signals. In order to determine whether decreased endothelial cell activation could reduce inflammation and neutrophil infiltration following acute myocardial infarction, the effects of MultiStem on reducing the inflammatory response induced by an ischemic event were examined in a rat model of AMI. Decreased levels of neutrophil infiltration were found, correlated with decreased levels of neutrophil tissue elastase in MultiStem treated hearts following permanent LAD ligation compared with vehicle treated controls.

### Materials and Methods

### Cell Culture

Human Aortic Endothelial Cells (HAEC) were purchased from Lonza (Walkersville, MD, http://www.lonza.com) and cultured according to manufacturer's instructions to passage 7 using Endothelial Growth Medium-2 (EGM-2) from Lonza. Human Pulmonary Microvascular Endothelial Cells (HPMEC) were purchased from ScienCell (Carlsbad, CA, www.sciencellonline.com) and cultured according to manufacturer's instructions to passage 4 using Endothelial Cell Medium (ECM) from ScienCell. Human Mesenchymal Stem Cells (MSC) were purchased from Lonza and cultured according to manufacturer's instructions to passage 6 using Mesenchymal Stem Cell Growth Medium (MSCGM) from Lonza. Human MultiStem was cultured as described¹⁸.

### Endothelial Co-culture Assay

Either HAEC or HPMEC were plated in the bottom of 6-well 0.4 µm membrane Transwell plates (Thermo Fisher Scientific, Waltham, MA, http://www.fishersci.com) at 1 x 10⁵ cells/cm² in their respective growth media and incubated for three days in a humidified, 5% CO₂, 37°C atmosphere. The media was aspirated and the cells rinsed with PBS. 2 ml of MultiStem® media was added per well and inserts containing 1:1, 1:4, 1:10, or 1:20 (HAEC: MultiStem). MultiStem in 1 ml of MultiStem media was placed in the wells. 10 ng/ml recombinant human Tumor Necrosis Factor alpha (rhTNFα) from Sigma-Aldrich (St. Louis, MO, http://www.sigmaaldrich.com) was added to each well. Controls consisting of endothelial cells alone (- rhTNFα), endothelial cells alone + 10 ng/ml rhTNFα, and 1:1 co-culture (- rhTNFα) were also set up. Controls were performed in 3 ml MultiStem media. The samples were then incubated for three days in a humidified, 5% CO₂, 37°C atmosphere. Alternatively, MSC and MSCGM were used in place of MultiStem and MultiStem media for comparative studies. The assay was also performed using 10 ng/ml recombinant human Interleukin 1 β (rhIL-1β) from Sigma-Aldrich in place of rhTNFα.

### Flow Cytometric Analysis

Cells were detached from the six well plates using 50/50 PBS/Enzyme Free (Millipore, Billerica, MA, http://www.millipore.com), spun down at 1600 rpm for 5 min and resuspended in 600 µl of PBS. Each sample was divided equally into three tubes for flow cytometric staining. The cells were incubated with 20 µl antibodies to either CD106 FITC conjugated (V-CAM1), CD54 PE conjugated (I-CAM1), or CD62E PE conjugated (E-Selectin) from BD Pharmingen (San Jose, CA, http://www.bdbiosciences.com) for 40 minutes at 4°C in the dark. Additionally, isotype controls were run using FITC Mouse IgG1 κ or PE Mouse IgG1 κ (BD Pharmingen) The cells were washed with 2 ml of PBS and spun down at 1600 rpm for 5 minutes. The supernatant was discarded and the cells resuspended in 200 µl of PBS + 1% paraformaldehyde (Electron Microscopy Sciences, Hatfield, PA, http://www.electronmicroscopysciences.com). Flow cytometric analysis was performed on a FACSVantage SE flow cytometer (Becton Dickinson Immunocytometry Systems, San Jose, California) equipped with a 488-nm argon laser. Emission from PE (FL2) fluorescence was detected using a 585/42 band pass filter. Data acquisition and analysis were performed using CellQuest™ software (Becton Dickinson).

### Immunofluorescence

12 mm Microscope cover glasses (Fisher Scientific) were placed in the bottom of the 6-well Transwell plates prior to plating the HAEC for the co-culture assay. The cells were blocked with 10% donkey serum (Jackson ImmunoResearch Laboratories, West Grove, PA, http://www.iacksonimmuno.com) for 1 hour at room temperature followed by an overnight incubation with a 1:50 dilution of mouse anti-human E-Selectin monoclonal antibody (Santa Cruz Biotechnology, Santa Cruz, CA, http://www.scbt.com) at 4°C with gentle agitation. The cells were then washed four times for five minutes each with PBS-T followed by incubation for one hour at room temperature with a 1:400 dilution of donkey anti-mouse AlexaFluor 488 (Invitrogen, Carlsbad, CA, http://www.invitrogen.com). The cells were then washed four times for five minutes each with PBS-T. Images were obtained at 100X magnification using a Leica microscope (Leica, Microsystems, Bannockburn, IL, http://www.leica-microsystems.com), Optronics camera and Magnafire software (Optronics, Goleta, CA, http://www.optronics.com).

### ELISA

The media was collected following the co-culture assay and two-fold diluted samples were used in an ELISA for soluble E-Selectin (R&D Systems, Minneapolis, MN, http://www.rndsystems.com) according to the manufacturer's instructions.

### RT-PCR

Cells were detached from the 6-well Transwell plates using 0.25% Trypsin-EDTA (Invitrogen), centrifuged, and resuspended in 1.8 ml lysis buffer. RNA was extracted using the Absolutely RNA Miniprep kit (Stratagene, La Jolla, CA, http://www.stratagene.com) according to the manufacturer's instructions. The RNA was eluted in 65 µl of the provided elution buffer. RT-PCR was performed using 100 pmol Random Primers (Promega, Madison, WI, http://www.promega.com). RT positive and RT negative as well as water were run on 96 well plates.

### Neutrophil Binding Assay

Neutrophils were isolated from peripheral blood from healthy volunteers. After isolation, neutrophils were activated for 10 minutes by the addition of LPS (0.1 µg/ml) for 10 minutes at 37 degrees. HAECs were incubated with or without TNF- , MultiStem or MSC as described above for 72 hours. The media was then removed, the cells were washed and endothelial cells were then incubated with activated neutrophils for 1 minute. The endothelial cells were then washed with PBS four times to remove any non-binding neutrophils. After multiple washes, neutrophil binding to endothelial cells was examined by microscopy (20x objective) and photographed. Neutrophils bound to the endothelial layer were counted in each field. Each conditioned was performed in triplicate and three fields were photographed per well.

### Animal Studies

Cell preparation - On each day of cell injections, MultiStem was thawed and tested for viability. Cells showing >90% viability were resuspended in PBS at 50 million cells/mL. Previous thawing experiments indicated that MultiStem cells remain >95% viable under these conditions for up to 8 hr.

Animal surgery - All experimental animal procedures described in this study were performed under the protocols approved by the Animal Research Committee of the Cleveland Clinic Foundation (Cleveland, Ohio, USA) Male Lewis rats, weighing between 150-175 g, were anesthetized with an i.p. injection of a mixture of ketamine (100mg/kg) and xylazine (5 mg/kg) and were ventilated with room air at 80 breaths/min (RSP1002, Kent Scientific Corp, Torrington, CT). Anterior wall myocardial infarction was induced in 25 rats by sternotomy and surgical ligation of the left anterior descending artery. Ligation of the LAD was confirmed by blanching of the tissue distal to the suture. Following LAD ligation, 10 million Lewis Rat MultiStem cells (50 million cells/ml) or PBS alone were injected into the heart in the area surrounding the infracted region. Animals were sacrificed three days following surgery. The hearts from 10 animals (5 vehicle treated and 5 MultiStem treated) were embedded in OCT and used for frozen thin sections. Sections were subsequently stained for elastase (to determine neutrophil counts)

### Statistics

Statistical analysis was performed using Student T-test or ANOVA with post-hoc analysis when appropriate with p < 0.05 accepted as statistically significant. Error bars are expressed as standard deviation.

### Results

### MultiStem prevents up regulation of cell adhesion molecules upon activation

Expression of adhesion molecules dramatically increases on the cell surface of endothelial cells upon exposure to pro-inflammatory cytokines such as TNF-α. To test whether MultiStem can modulate this response, MultiStem was co-cultured in transwells with human aortic endothelial cells (HAECs) in the presence or absence of TNF- α for three days. Subsequently, the cell surface expression of E-selectin, V-CAM and I-CAM was measured by FACs analysis or immunostaining (Figure 1A). After 72 hours, TNF- α induced high levels of all three markers in the absence of MultiStem compared to unactivated controls. We found, however, that in the presence of MultiStem, the number of cells with surface expression of E-selectin (Figure 1B)(p=0.0149) and V-CAM (p=0.0037) were significantly reduced. (Figure 1B) Additionally, the level of cell surface expression of I-CAM (p =0.001) was reduced at the highest doses of MultiStem (Figure 1C). The modulation of cell surface adhesion molecule up regulation by MultiStem is cell dose dependent, is reduced with decreasing concentrations of MultiStem and is reproducible with MultiStem derived from different donors (data not shown). To examine whether this effect was specific to TNF- α, this experiment was repeated using interleukin 1β (Il-1β). Similar to the results with TNF-α, co-culture with MultiStem inhibited upregulation of adhesion molecule (E-selectin, V-CAM and I-CAM) to the cell surface when activated with Il-1β compare to controls (Figure 2B) .

Next, it was determined whether downregulation of surface expression of cell adhesion molecules in the presence MultiStem was an aortic endothelial specific response or if other endothelial cell lines also responded to MultiStem in a similar manner. Human pulmonary microvascular endothelial cells (HPMECs) were tested to see if these cells downregulated TNF-α induced cell adhesion molecules cell surface expression in response to MultiStem in a manner similar to HAECS (Figure 2A). The number of HPMECs expressing TNF-α induced E-selectin (p<0.0001) and V-CAM (p<0.0001) on their cell surface was reduced after co-culture with MultiStem. Similarly, I-CAM cell surface expression was also significantly reduced in cells co-cultured with the highest dose of MultiStem (p =0.028) (Figure 2A). Since these experiments were performed in transwells, direct interaction between MultiStem and endothelial cells is not required for MultiStem to modulate endothelial cell adhesion marker activation. These data therefore suggest that soluble factors secreted from MultiStem act in a paracrine manner to reduce endothelial surface adhesion molecule expression.

### MultiStem does not increase shedding of adhesion molecules but rather functions by preventing an induction of adhesion molecule RNA expression

One possible mechanism by which MultiStem could decrease cell surface expression of adhesion molecules such E-selectin is to increase cleavage of those molecules from the cell surface. Cleavage of adhesion molecules from the cell surface is mediated by different proteases including metalloproteases such as TACE/ADAM17. Previous studies suggested that soluble adhesion molecules may be pathological and can contribute to neutrophil activation^{19, 20}. Treatment with MultiStem was examined to determine if it decreases E-selectin surface expression by increasing E-Selectin surface shedding. The levels of soluble E-selectin secreted into the conditioned media by endothelial cells in the presence or absence of MultiStem were measured by ELISA. No increase was found in sE-selectin concentration in conditioned media collected from co-cultures of MultiStem with either HAECs or HPMECS (Figure 3A, data not shown). Conversely, a decrease in soluble E-selectin was found in the media from TNF- α activated HAECs (p < 0.001) and HPMECs (p <0.001) co-cultured with MultiStem at the highest ratios, suggesting that the decrease in surface expression of E-selectin is a consequence of decreased surface expression with subsequent reduction in the shedding of soluble E-Selectin (Figure 3A). Therefore, the decrease in surface expression of E-selectin is not due to increased cleavage of this molecule from the cell surface, implying that the decrease in cell adhesion molecules on the surface of endothelial cells is not a result of proteolytic cleavage of these molecules.

Since cell surface adhesion molecules E-selectin, V-CAM and I-CAM are all transcriptionally activated in endothelial cells when treated with pro-inflammatory cytokines such as TNF-α, it was determined whether MultiStem could modulate the regulation of these adhesion molecules at the transcriptional level (Figure 3). Endothelial cells (HAECS) were co-cultured with MultiStem in the presence or absence of TNF-α for 72 hours. RNA was subsequently isolated from the endothelial cells and reverse transcribed into cDNA. Quantitative PCR was then performed using primers for E-selectin, V-CAM, I-CAM and GAPDH. Expression levels of mRNA were all normalized to GAPDH levels (Figure 3B). These results indicate that E-selectin, V-CAM, and I-CAM mRNA levels are reduced in the presence of MultiStem, suggesting that MultiStem inhibits TNF-α upregulation of these genes. Similar results were seen when MultiStem was co-cultured with pulmonary endothelial cells (HPMECs, data not shown). V-CAM, I-CAM and E-selectin transcriptional activation by TNF-α is NF- B dependent, suggesting that MultiStem may be modulating NF- B signaling at some level.

### Unlike MultiStem. MSC do not significantly modulate cell adhesion molecule expression

Other bone marrow derived stem cells have also been shown to modulate immune function by preventing T-cell proliferation, suppressing natural killer cell function and modulating immune disorders such as Crohn's disease and GVHD in animal models. In order to assess whether the immunomodulatory effects of MultiStem on endothelial cell activation were also produced by other stem cell lines, endothelial cells were co-cultured with MSC in the presence of TNF- α for 72 hours and cell adhesion molecule cell surface expression was subsequently examined. Surprisingly, TNF-α induced endothelial cell adhesion molecule surface expression is unchanged in the presence of MSC compared to untreated controls, whereas MultiStem significantly reduces adhesion molecule expression (Figure 4A-C). A slight decrease in E-selectin surface expression was observed with MSC at the highest dose, however, this change was significantly smaller than the reduction seen in the presence of MultiStem (Figure 4A). Therefore, MSC do not modulate adhesion molecule upregulation by TNF-α. These results show that MultiStem has a functionally different secretion profile from MSC that is reflected in differences in their biological activity.

### Co-culture of activated endothelial cells with MultiStem prevents neutrophil binding

Upregulation of cell adhesion molecules on the cell surface of endothelial cells have been shown to be critical to rolling and firm adhesion of immune cells such as neutrophils⁴. In order to determine whether the modulation of adhesion molecule upregulation to the cell surface by MultiStem is sufficient to affect neutrophil binding to endothelial cells, neutrophil binding to MultiStem treated endothelial cells was compared to untreated endothelial cells. Endothelial cells were grown to a confluent monolayer, treated with TNF-α for 72 hours in the presence or absence of MultiStem. Neutrophils were isolated from at least 2 different donors, activated by the addition of LPS (0.1µg/ml) and incubated with endothelial cells for one minute. After multiple washes, endothelial cell and neutrophil binding was examined by microscopy (10x objective) and photographed. Neutrophils bound to the endothelial layer were counted in each field (Figure 5A). Only low levels of activated or unactivated neutrophils bound to unactivated endothelial cells. However, upon activation with TNF-α, a significant increase was seen in bound neutrophils, as expected (Figure 5B). When endothelial cells were preincubated with MultiStem, neutrophil binding was significantly reduced (p < .0001). These results demonstrate that the change in adhesion molecule expression on endothelial cells is sufficient to change the binding characteristics of endothelial cell layer to neutrophils. In contrast, co-culture of MSC with the endothelial cells during the TNF-α activation did not alter neutrophil binding. Additionally, when TNF-α activated endothelial cells, alone or in co-culture with MSC, were exposed to neutrophils, there was a significant change in endothelial cell morphology to a longer thinner cell type and a decrease in cell-cell contact (Figure 5C). In endothelial cells co-cultured with MultiStem when TNF-α activated, the cell morphology does not change as much and cell-cell contact is maintained. These data suggest that MultiStem functionally alters activated endothelial cells. Therefore, MultiStem was tested to determine whether it could affect neutrophil migration *in vivo.*

### Treatment with MultiStem decreases neutrophil infiltration following LAD-induced acute myocardial infarction

Previous animal studies have shown that direct injection of MultiStem into peri-infarct sites following myocardial infarction, induced by direct left anterior descending ligation, results in improved cardiac function^{14, 16}. The results in this report show that MultiStem can downregulate cell surface expression of adhesion molecules (E-selectin, V-CAM and I-CAM) in the presence of TNF-α. These molecules are critical for neutrophil adhesion and extravasation through the endothelium. Since neutrophil infiltration into the heart after ischemia can be deleterious and can result in decreased cardiac function following an ischemic insult^{10, 11}, it was hypothesized that improvement in heart function after MultiStem treatment was due, in part, to reduced inflammation and neutrophil infiltration into the peri-infract zone following AMI. To test this hypothesis, the levels of neutrophils present in the heart were examined following induced myocardial infarction in rats.

Anterior wall myocardial infarction was induced in rats by ligation of the left anterior descending artery (LAD ligation). Following LAD ligation, 10 million Lewis Rat MultiStem cells or PBS alone were injected into the heart in the area surrounding the infarcted region. Animals were sacrificed three days following surgery, when the inflammatory response is at its peak. The hearts from 10 animals (5 vehicle treated and 5 MultiStem treated) were sectioned and stained for elastase to determine the extent of neutrophil infiltration. Neutrophil infiltration was examined within the peri-infarct zone (within 0.25-0.5 cm of infarcted tissue) for both treated and untreated animals (Figure 6).

At three days post AMI and cell treatment, MultiStem treated animals exhibited significantly less neutrophil infiltration into the peri-infarct area, compared to vehicle treated controls (12.185 neutrophils per high powered field (Hpf) versus 35.25 neutrophils/Hpf, p=.005823) (Figure 6 A-C). Elastase positive cells were counted in four high powered fields per animal and averaged (Figure 6 B,C). These results confirm that MultiStem has an anti-inflammatory effect on the heart following AMI. These data also suggest that the decrease observed in endothelial cell adhesion molecules upregulation upon TNF-α activation in culture may translate *in vivo* into a functional change of the endothelial cell barrier in the presence of MultiStem.

### Conclusions

In this study, MultiStem was examined to determine whether it could modulate activation of endothelial cells in response to inflammatory stimuli. MultiStem are large-scale expanded clinical grade multipotent progenitor cells (MAPC). The results show that both aortic and pulmonary endothelial cells have reduced cell surface expression of adhesion molecules E-selectin, V-CAM and I-CAM when activated with TNF-α or IL-1β when co-cultured with MultiStem compared to untreated controls. Additionally, the experiments demonstrate that the decrease in E-selectin expression is not due to reduced shedding of this molecule from the cell surface. Rather, V-CAM, E-selectin and I-CAM mRNA expression is reduced compared to TNF-α treated endothelial cells alone, suggesting that MultiStem is modulating the increased transcription of these proteins in response to inflammatory signals. Furthermore, it was observed that neutrophil adhesion to activated endothelial cells was also decreased when endothelial cells were incubated with MultiStem during TNF-α activation. In order to investigate whether these changes were relevant *in vivo,* treatment with MultiStem was examined to determine if it would modulate inflammation after ischemic injury. Indeed, neutrophil infiltration was reduced in the peri-infarct in MultiStem treated animals three days following AMI in rats compared with vehicle controls.

The molecular mechanism responsible for the downregulation the cell adhesion molecules on activated endothelial cells in the presence of MultiStem has not yet been characterized. However, since the co-culture studies were performed in transwells, MultiStem must be secreting one or more soluble factors into the media which subsequently acts on endothelial cells to prevent or downregulate the transcription of these integrins and selectins. Upon binding of cytokines such as TNF-α or IL-1β to their receptors, initiation of internal signaling results in NF-κB signaling dependent transcription. Transcription of E-selectin, I-CAM and V-CAM are all NF-κB dependent, suggesting that MultiStem may modulate NF-κB signaling.

Although MSCs have been shown to have immunomodulatory properties in other settings, MSCs did not prevent cell adhesion molecule upregulation in activated endothelial cells when co-cultured with these cells. V-CAM and I-CAM expression did not exhibit any downregulation upon co-culture with any dose of MSC. At the highest dose of MSC, E-selectin was modestly reduced from the cell surface compared to the untreated controls. However, E-selectin surface expression was significantly higher than when endothelial cells were co-cultured with MultiStem. Additionally, no effect was observed on E-selectin when activated endothelial cells were co-cultured with MSCs at lower doses. These results have a few implications. First, the mechanism utilized by MSC to modulate T-cell proliferation is different than the mechanism utilized by MultiStem to downregulate endothelial cell surface markers, suggesting that stem cells can modulate immune function through multiple pathways. Second, these results imply that MultiStem and MSCs have distinct secretion profiles which are reflected in the differences in the functional activities of these two cell types. These differences may inform which cell type may be best to potentially treat various clinical indications, with each cell type best suited for use in a different class of indications. Further research into the differences between these stem cell types and their mechanisms of action will help in clarifying these issues.

Although the effects of MultiStem on endothelial cells have been examined in the context of AMI, adhesion of lymphocytes and leukocytes to the endothelium is a critical part of the inflammatory process and therefore, deleterious in many disorders, including other ischemic injuries, such as stroke, as well as immune disorders such as GVHD, acute respiratory distress syndrome (ARDS), and multiple sclerosis. The results show that MultiStem modulates cell adhesion molecule upregulation in at least two endothelial cell types, aortic and pulmonary upon activation with two different cytokines, TNF-α and Il-1β. This study suggests that the immunomodulatory activity of MultiStem extends to multiple endothelial cell types and inflammatory signals. Thus, treatment with MultiStem may confer benefit in disorders in which infiltration of immune cells through the epithelium results in increased injury and damage.

### References

1. Wagner et al. The vessel wall and its interactions. Blood. Jun 1 2008;111(11):5271-5281.
2. Golias C, Tsoutsi E, Matziridis A, Makridis P, Batistatou A, Charalabopoulos K. Review. Leukocyte and endothelial cell adhesion molecules in inflammation focusing on inflammatory heart disease. In vivo (Athens, Greece). Sep-Oct 2007;21(5):757-769.
3. Bullard et al., Infectious susceptibility and severe deficiency of leukocyte rolling and recruitment in E-selectin and P-selectin double mutant mice. The Journal of experimental medicine. May 1 1996;183(5):2329-2336.
4. Rao et al., Endothelial-dependent mechanisms of leukocyte recruitment to the vascular wall. Circulation research. Aug 3 2007;101(3):234-247.
5. Frenette et al., Susceptibility to infection and altered hematopoiesis in mice deficient in both P- and E-selectins. Cell. Feb 23 1996;84(4):563-574.
6. Furman et al., Effect of elevated leukocyte count on in-hospital mortality following acute myocardial infarction. The American journal of cardiology. Oct 15 1996;78(8):945-948.
7. Menon et al., Leukocytosis and adverse hospital outcomes after acute myocardial infarction. The American journal of cardiology. Aug 15 2003;92(4):368-372.
8. Takahashi et al., Relationship of admission neutrophil count to microvascular injury, left ventricular dilation, and long-term outcome in patients treated with primary angioplasty for acute myocardial infarction. Circ J. Jun 2008;72(6):867-872.
9. Gonon et al., Limitation of infarct size and attenuation of myeloperoxidase activity by an endothelin A receptor antagonist following ischaemia and reperfusion. Basic Res Cardiol. Sep 2001;96(5):454-462.
10. Vasilyev et al., Myeloperoxidase-generated oxidants modulate left ventricular remodeling but not infarct size after myocardial infarction. Circulation. Nov 1 2005;112(18):2812-2820.
11. Vinten-Johansen J., Involvement of neutrophils in the pathogenesis of lethal myocardial reperfusion injury. Cardiovasc Res. Feb 15 2004;61(3):481-497.
12. Cheng et al., TH1/TH2 functional imbalance after acute myocardial infarction: coronary arterial inflammation or myocardial inflammation. Journal of clinical immunology. May 2005;25(3):246-253.
13. Kovacsovics-Bankowski et al., Clinical scale expanded adult pluripotent stem cells prevent graft-versus-host disease. Cellular immunology. 2009;255(1-2):55-60.
14. Van't Hof et al., Direct delivery of syngeneic and allogeneic large-scale expanded multipotent adult progenitor cells improves cardiac function after myocardial infarct. Cytotherapy. 2007;9(5):477-487.
15. Kovacsovics-Bankowski et al., Pre-clinical safety testing supporting clinical use of allogeneic multipotent adult progenitor cells. Cytotherapy. 2008;10(7):730-742.
16. Pelacho et al., Multipotent adult progenitor cell transplantation increases vascularity and improves left ventricular function after myocardial infarction. Journal of tissue engineering and regenerative medicine. Jan-Feb 2007;1(1):51-59.
17. Zeng et al., Bioenergetic and functional consequences of bone marrow derived multipotent progenitor cell transplantation in hearts with postinfarction LV remodeling. Circulation. 2007;In press.
18. Perry et al., Clinical Scale Expansion of Human Pluripotent Stem Cells. ASH Annual Meeting Abstracts. November 16, 2005 2005;106(11):1060-.
19. Vainerc et al., Serum Concentration and Chemotactic Activity of E-selectin (CD62E) in Inflammatory Bowel Disease. Mediators of inflammation. 1994;3(3):215-218.
20. Zeitler et al., Elevated serum concentrations of soluble adhesion molecules in coronary artery disease and acute myocardial infarction. European journal of medical research. Sep 29 1997;2(9):389-394.
21. Lloyd-Jones et al., Heart Disease and Stroke Statistics--2010 Update. A Report From the American Heart Association. Circulation. Dec 17 2009.
22. Aranguren et al., Multipotent adult progentor cells sustain function of ischemic limbs by stimulating vessel and muscle regeneration. 2007.
23. Wragg et al., VEGFR1/CXCR4 positive progenitor cells modulate local inflammation and augment tissue perfusion by a SDF1 dependent mechanism. The Journal of clinical investigation. 2006.
24. Hare et al., A randomized, double-blind, placebo-controlled, dose-escalation study of intravenous adult human mesenchymal stem cells (prochymal) after acute myocardial infarction. Journal of the American College of Cardiology. Dec 8 2009;54(24):2277-2286.
25. Brenneman et al., Autologous bone marrow mononuclear cells enhance recovery after acute ischemic stroke in young and middle-aged rats. J Cereb Blood Flow Metab. Jan;30(1): 140-149.
26. Halkos et al., Intravenous infusion of mesenchymal stem cells enhances regional perfusion and improves ventricular function in a porcine model of myocardial infarction. Basic Res Cardiol. Nov 2008;103(6):525-536.
27. Tang et al., Mesenchymal stem cells participate in angiogenesis and improve heart function in rat model of myocardial ischemia with reperfusion. Eur J Cardiothorac Surg. Aug 2006;30(2):353-361.
28. Ringden et al., Mesenchymal stem cells for treatment of therapy-resistant graft-versus-host disease. Transplantation. May 27 2006;81(10):1390-1397.
29. Ringden et al., Mesenchymal stem cells combined with cyclosporine inhibits cytotoxic T cells. Biol Blood Marrow Transplant. Jun 2006;12(6):693-694.
30. Highfill et al., Multipotent adult progenitor cells (MAPC) can suppress graft-versus-host disease via prostaglandin E2 synthesis and only if localized to sites of allopriming. Blood. May 20 2009.

### Example 2

### Increasing Potency for Downregulation

The endothelial cell assay has been used to identify a treatment regiment of MultiStem that mimics coculture of MultiStem with activated endothelial cells or activated T-cells. Previously, the inventors had found that coculture of MultiStem with activated endothelial cells was required to induce the anti-inflammatory activity of MultiStem in the endothelial cell assay (downregulation of E-Selectin, I-CAM and V-CAM). In other words, conditioned media collected from MultiStem grown under its normal culture conditions was insufficient to downregulate endothelial cell adhesion molecules after activation with TNF-a or cytomix. However, the inventors performed an experiment in which they treated MultiStem for 3 days with "cytomix" (a mixture of 10ng/ml of each TNF-α, Interferon-gamma and Interluekin 1 beta). They subsequently collected the conditioned media from MultiStem after this treatment. This conditioned media was then used as a substitute for MultiStem in the endothelial cell assay (it was added to activated endothelial cells with either TNF-a or cytomix and cultured for 2 or 3 days). They found that unlike unconditioned basal media or media collected from MultiStem cultured under normal conditions, this "cytomix" treated media was sufficient to prevent endothelial cell adhesion molecule upregulation. This media could substitute for coculture of MultiStem with the activated endothelial cells. They subsequently utilized this media to test if it could substitute for coculture of MultiStem with T-cell in the T-cell proliferation assay and found that it was sufficient to prevent T-Cell proliferation induced by CD3/CD28, unlike conditioned media from untreated MultiStem.

Therefore, they have concluded that treatment of MultiStem with cytomix is sufficient to induce the anti-inflammatory activity of MultiStem required to prevent T-Cell activation and endothelial cell adhesion molecule upregulation. This was discovered using the endothelial cell assay.

Preferred embodiments of the invention are as follows:
1. A method of obtaining cells with a desired potency for one or more of the following: (1) reduce leukocyte extravasation, (2) reduce leukocyte adhesion to vascular endothelium or to isolated endothelial cells, (3) reduce cytokine-mediated activation of endothelial cells, (4) reduce expression of one or more cell adhesion molecules on an endothelial cell, said method comprising assessing cells for and selecting cells with a desired potency for one or more of the effects of (1) - (4) above, the cells being non-embryonic, non-germ cells that express one or more of oct4, telomerase, rex-1, or rox-1 and/or can differentiate into cell types of at least two of endodermal, ectodermal, and mesodermal germ layers.
2. A method to treat inflammation in a subject, the method comprising administering the selected cells of embodiment 1 to the subject in a therapeutically effective amount and for a time sufficient to achieve a therapeutic result.
3. A method to construct a cell bank, the method comprising expanding and storing the selected cells of embodiment 1 for future administration to a subject.
4. A method for drug discovery, the method comprising contacting the selected cells of embodiment 1 with an agent to assess the effect of the agent on the ability of the cells to effect any of events (1)-(4).
5. A composition comprising cells assessed and selected for the desired potency to achieve one or more of the following: 1) reduce leukocyte extravasation, (2) reduce leukocyte adhesion to vascular endothelium or to isolated endothelial cells, (3) reduce cytokine-mediated activation of endothelial cells, (4) reduce expression of one or more cell adhesion molecules on an endothelial cell; and
   the cells being non-embryonic, non-germ cells that express one or more of oct4, telomerase, rex-1, or rox-1 and/or can differentiate into cell types of at least two of endodermal, ectodermal, and mesodermal germ layers.
6. The method of any of embodiments 1-5 wherein the cytokine is selected from the group consisting of TNF-α and IL-1.
7. The method of any of embodiments 1-5 wherein cytokine-mediated activation produces an increase in expression of one or more of E-selectin, P-selectin, VCAM-1, ICAM, and VCAM-2 in the endothelial cell.
8. The methods of any of embodiments 1-5 wherein the cell adhesion molecule, the expression of which is reduced in the endothelial cell, is one or more of E-selectin, P-selectin, VCAM-1, ICAM, and VCAM-2.
9. A method to increase the potency of a cell to effect one of more of the following: 1) reduce leukocyte extravasation, (2) reduce leukocyte adhesion to vascular endothelium or to isolated endothelial cells, (3) reduce cytokine-mediated activation of endothelial cells, (4) reduce expression of one or more cell adhesion molecules on an endothelial cell; the method comprising contacting cells with IFN-γ, IL-1β, and TNF-α to produce cells that achieve one or more of effects (1) - (4);
   the cells being non-embryonic, non-germ cells that express one or more of oct4, telomerase, rex-1, or rox-1 and/or can differentiate into cell types of at least two of endodermal, ectodermal, and mesodermal germ layers.

## Claims

1. Cells for use in treating inflammation in a subject, wherein the cells have a desired potency for one or both of the following: (1) reduce cytokine-mediated activation of endothelial cells and (2) reduce expression of one or more cell adhesion molecules on an endothelial cell, the cells being non-embryonic, non-germ cells that express telomerase, have a normal karyotype and are not tumorigenic, and wherein, prior to administration of the cells to the subject, the cells are assayed for having the desired potency.

2. The cells for use of claim 1 wherein the cells are allogeneic.

3. The cells for use of any one of claims 1 or 2 wherein the subject is human.

4. An *in vitro* method of (1) reducing cytokine-mediated activation of endothelial cells and/or (2) reducing expression of one or more cell adhesion molecules on an endothelial cell, said method comprising exposing endothelial cells to non-embryonic, non-germ cells that express telomerase, have a normal karyotype and are not tumorigenic, and assaying the endothelial cells for (1) reduced cytokine-mediated activation and/or (2) reduced expression of one or more cell adhesion molecules.

5. A method to increase the potency of cells to: (1) reduce cytokine-mediated activation of endothelial cells and/or (2) reduce expression of one or more cell adhesion molecules on an endothelial cell, the method comprising contacting cells with IFN-γ, IL-1β, and TNF-α to produce cells that achieve one or more of effects (1)-(2);
the cells being non-embryonic, non-germ cells that express telomerase, have a normal karyotype and are not tumorigenic.

6. The cells produced by the method to increase potency according to claim 5 for use in treating inflammation in a subject.

7. A method for constructing a cell bank, the method comprising expanding and storing, for future administration to a subject, cells produced by the method to increase potency according to claim 2.

8. A method for constructing a cell bank, the method comprising expanding and storing, for future administration to a subject, cells that have a desired potency for one or both of the following: (1) reduce cytokine-mediated activation of endothelial cells and (2) reduce expression of one or more cell adhesion molecules on an endothelial cell, the cells being non-embryonic, non-germ cells that express telomerase, have a normal karyotype and are not tumorigenic, wherein the cells are assayed for having the desired potency.

9. A method for drug discovery, the method comprising exposing *in vitro,* cells that have a desired potency for one or both of the following: (1) reduce cytokine-mediated activation of endothelial cells and (2) reduce expression of one or more cell adhesion molecules on an endothelial cell to an agent to assess the effect of the agent on the ability of the cells to effect one or both of (1)-(2) above, the cells being non-embryonic, non-germ cells that express telomerase, have a normal karyotype and are not tumorigenic, wherein, prior to exposing *in vitro,* the cells are assayed for having the desired potency.

10. The cells for use of any of claims 1-3 or the method of any of claims 4-9 wherein the cytokine is selected from the group consisting of TNF-α and IL-1.

11. The cells for use of any of claims 1-3 or the method of any of claims 4-10 wherein cytokine-mediated activation produces an increase in expression of one or more of E-selectin, P-selectin, VCAM-1, ICAM, and VCAM-2 in the endothelial cell.

12. The cells for use of any of claims 1-3 or the method of any of claims 4-11 wherein the cell adhesion molecule, the expression of which is reduced in the endothelial cell, is one or more of E-selectin, P-selectin, VCAM-1, ICAM and VCAM-2.

13. The cells for use of any of claims 1-3 or the method of any of claims 4-12 wherein the cells express Oct-4.

14. The cells for use of any of claims 1-3 or the method of any of claims 4-13 wherein the cells express rex-1 and rox-1.

15. The cells for use of any of claims 1-3 or the method of any of claims 4-14 wherein the cells can differentiate into cell types of at least two of ectodermal, endodermal, and mesodermal germ layers.

16. The cells for use of any of claims 1-3 or the method of any of claims 4-15 wherein the cells can differentiate into cell types of ectodermal, endodermal, and mesodermal germ layers.

17. The cells for use of any of claims 1-3 or the method of any of claims 4-16 wherein the cells are derived from human bone marrow.
